# EUROPEAN PATENT SPECIFICATION

(11) **EP 1 670 809 B1**
(45) Date of publication and mention of the grant of the patent: **13.01.2016**
(21) Application number: 04784157.2
(22) Date of filing: 15.09.2004
(51) Int. Cl.: C07H 19/00, C07H 19/16, C07H 19/22

(54) **SYNTHESIS AND MANUFACTURE OF PENTOSTATIN AND ITS PRECURSORS, ANALOGS AND DERIVATIVES**
SYNTHESE UND HERSTELLUNG VON PENTOSTATIN UND VORSTUFEN, ANALOGA UND DERIVATEN DAVON
SYNTHESE ET PRODUCTION DE PENTOSTATINE, PRECURSEURS, ANALOGUES ET DERIVES DE CETTE DERNIERE

(30) Priority: 15.09.2003 US 503237 P
(43) Date of publication of application: 21.06.2006
(73) Proprietor: Mayne Pharma plc, Leamington Spa CV32 3RW (GB)
(72) Inventor: PHIASIVONGSA, Pasit, Brentwood, CA 94513 (US); REDKAR, Sanjeev, Hayward, CA 94545 (US)
(74) Representative: Modiano, Micaela Nadia
(86) International application number: PCT/US2004/030203
(87) International publication number: WO 2005/027838

(56) References cited:
- FR-A- 2 411 191
- US-A- 4 014 769
- US-A- 4 014 769
- US-A- 4 935 505
- US-A- 5 831 072

## Description

### BACKGROUND OF THE INVENTION

### Field of the Invention

The present invention relates to compositions and methods for preparing and manufacturing pentostatin ((8***R***)-3-(2-deoxy-β-D-*erythro*-pentofuranosyl)-3,6,7,8-tetrahydroimidazo[4,5-*d*][1,3]diazepin-8-ol), precursors of pentostatin, pentostatin analogs and derivatives, and other heterocycles that require expansion of the heterocyclic ring at an O-C-N functionality.

### Description of Related Art

Pentostatin, (8***R***)-3-(2-deoxy-β-d-*erythro*-pentofuranosyl)-3,6,7,8-tetrahydroimidazo[4,5-*d*][1,3]diazepin-8-ol,is a potent the potent inhibitor of adenosine deaminase. The chemical structure of pentostatin is shown below:

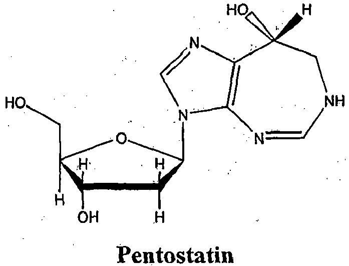

The total synthesis of pentostatin poses a challenge since the molecule contains (1) a unique and unstable heterocyclic base, (2) a 2-deoxy sugar that defies attempts at stereocontrolled glycosylation to favor the β-anomer, and (3) a central chiral hydroxyl group. The merit of any chemical transformation is measured by its resolutions to these three key difficulties.

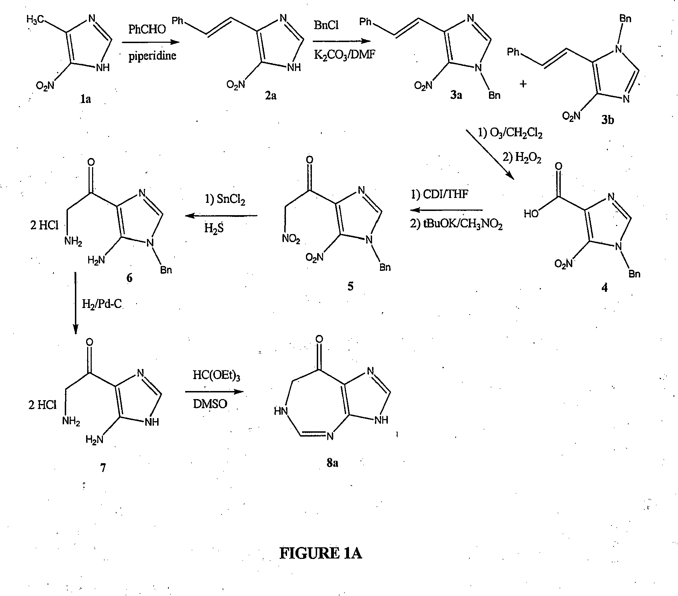

The first synthesis of Pentostatin was demonstrated by Showalter and Baker of Warner-Lambert/Parke-Davis Pharmaceutical. Chan, E.; Putt, S. R.; Showalter, H. D. H.; Baker, D. C. J. Org. Chem. 1982, 47, 3457-3464. The procedure focused mainly on the synthesis of the heterocyclic base (**Figures 1A-C**). The crux of the procedure is synthesis of diamine precursor such as **7**, which can have a ketone function (as shown) or a chiral alcohol group in the position α to the imidazole. The latter proposal has yet to be realized. Certain aspects of this approach make it flexible and amenable to improvement. Nevertheless, the procedure requires no less than 8 steps to synthesize just the base precursor **8a.** The chemical structure of the base **precursor 8a** is shown below. Such a procedure is difficult to commercially reduce to practice and expensive to scale-up to manufacturing size. Column chromatography is required for purification at many of these steps.

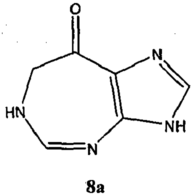

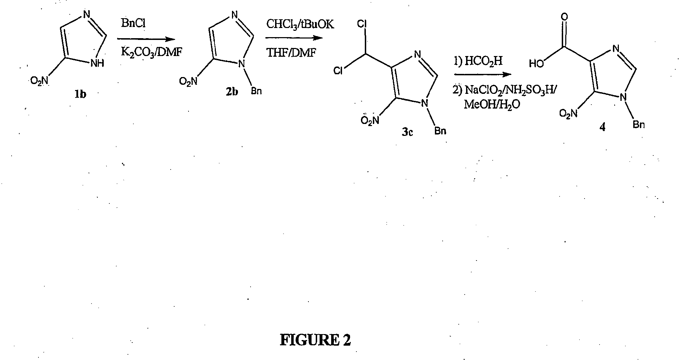

To further improve efficiency and minimize cost for manufacturing synthetic pentostatin, Chen et al. synthesized precursor **4** from a different starting material (**Figure 2**). Chen, B.-C.; Chao, S. T.; Sundeen, J. E.; Tellew, J.; Ahmad, S. Tetrahedron Lett. 2002, 43, 1595-1596. The modifications eliminated the N-2 benzylation side-reaction (no formation of **3b**), improved total yield of precursor 7 from 19% to 30%, and used less expensive starting material **1b**.

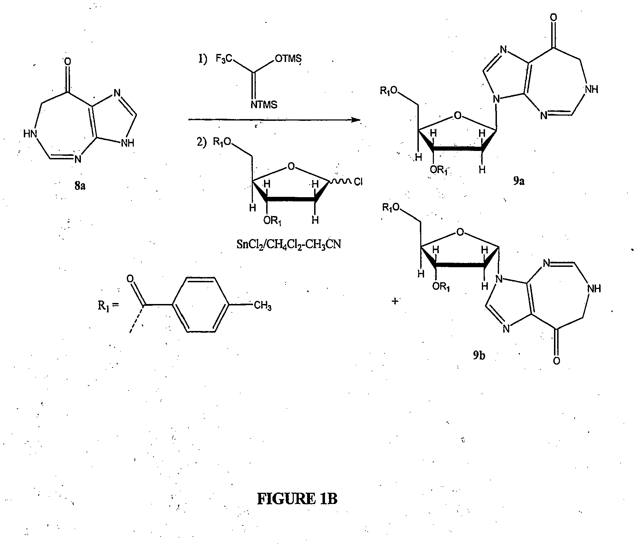

Of the numerous methods available for glycosylation, once precursor **8a** has been synthesized and purified, Showalter and Baker condensed it to the 2-deoxy sugar via a peracylglycosyl chloride adapted from the stannic chloride catalyzed process of Vorbrüggen (**Figure 1B**) to generate two anomers of pentostatin precursors **9a** and **9b**. The chemical structures of the pentostatin precursors **9a** and **9b** are shown below. 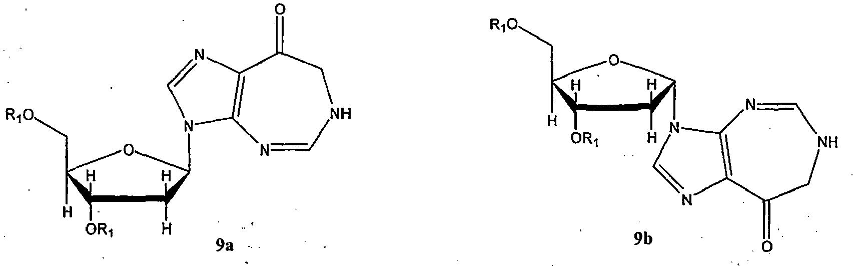 Chan, E.; Putt, S. R.; Showalter, H. D. H.; Baker, D. C. J. Org. Chem. 1982, 47, 3457-3464 . There was no stereocontrol of the glycosylation, but the 1:1 anomeric mixture could be separated by traditional column chromatography or fractional crystallization. In addition to the multi-steps route shown in **Figure 1A**, the peracylglycosyl chloride starting material must also be prepared by a multi-step procedure, which as a whole added to an already lengthy procedure that required isolation and purification steps.

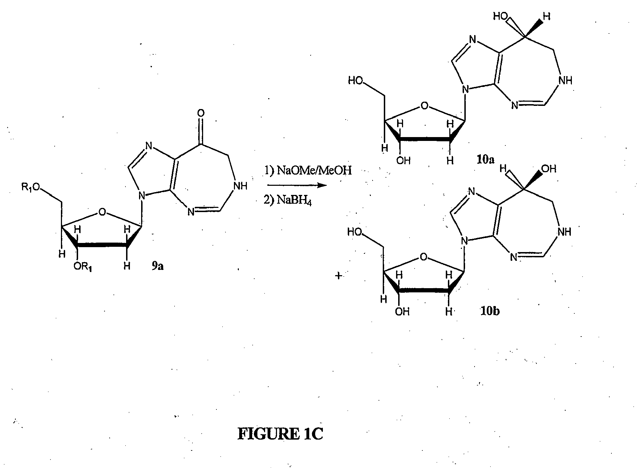

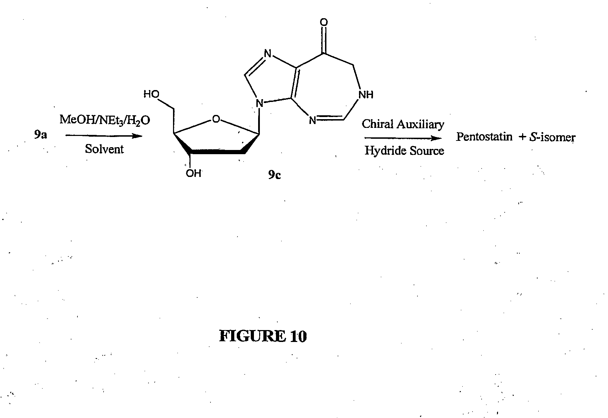

After **9a** (23%) had been isolated in pure form the protective group was removed and subsequently reduced with sodium borohydride to pentostatin (**Figure 1C**, **10a**), which converted the carbonyl functionality into a chiral hydroxyl group. However, since the transformation transpired without stereocontrol, a diastereomeric mixture of compounds **10a** and **10b** was obtained. The chemical structures of compounds **10a** and **10b** are shown below.

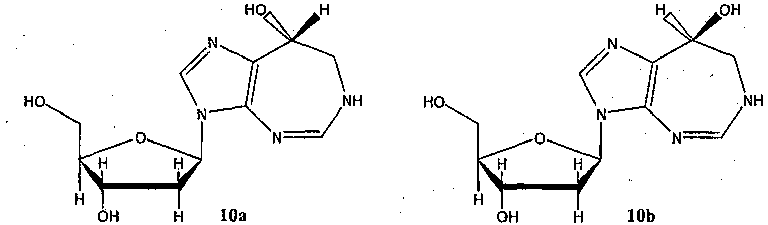

Various sterically hindered borohydrides (potassium tri-*sec*-butylborohydride and 9-borabicyclo[3.3.1]nonane; lithium tri-*tert*-butoxyaluminum hydride, lithium aluminum hydride-(-)-menthol complex, lithium aluminum hydride-(-)-*N*-methylephedrine-3,5-xylenol complex) were considered, but they found little improvement in enantio-selectivity or yield. Chan, E.; Putt, S. R.; Showalter, H. D. H.; Baker, D. C. J. Org. Chem. 1982, 47, 3457-3464. Separation of the 1:1 mixture of diastereomers **10a** (33%) and **10b** (29%) were determined best by a C-18 reverse-phase preparative HPLC for small scales. For larger scales, fractional crystallization was better.

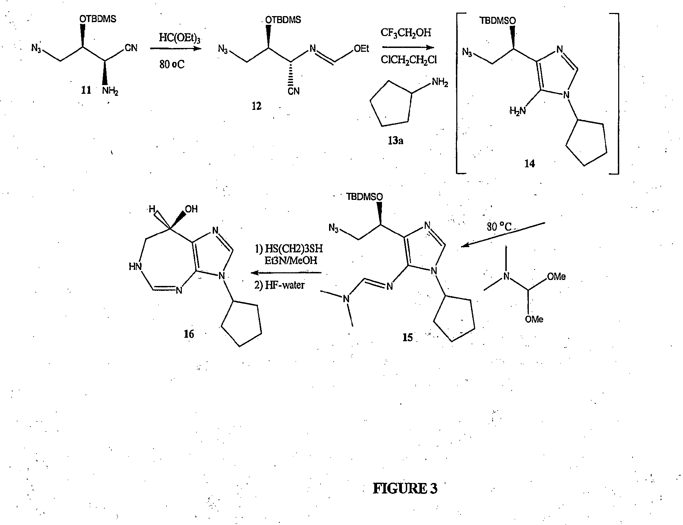

Another approach to resolving the three key difficulties was proposed by Rapoport (Ho, J. Z.; Mohareb, R. M.; Ahn, J. H.; Sim, T. B.; Rapoport, H. J. Org. Chem. 2003, 68, 109-114). This approach involved enantiocontrolled synthesis of the base with the natural *R* configuration of the hydroxyl group in place. To illustrate this approach, analogues of pentostatin were synthesized (**Figure 3**; cyclopentyl analogue). First precursor **11** was obtained by a multi-steps procedure starting with L-methionine, which required at least 8 synthetic steps. Truong, T. V.; Rapoport, H. J. Org. Chem. 1993, 58, 6090-6096. However, synthesis of pentostatin itself has yet to be realized, which not only would have a lengthier process but also unresolved stereo-chemical difficulties with the sugar moiety.

The approach proposed by Rapoport is very promising. It resolves a key stereochemical difficulty by incorporating a carefully designed synthetic pathway. The one serious drawback is that it still involves several synthetic steps. Nevertheless, it more than matches the synthetic route proposed by Showalter and Baker.

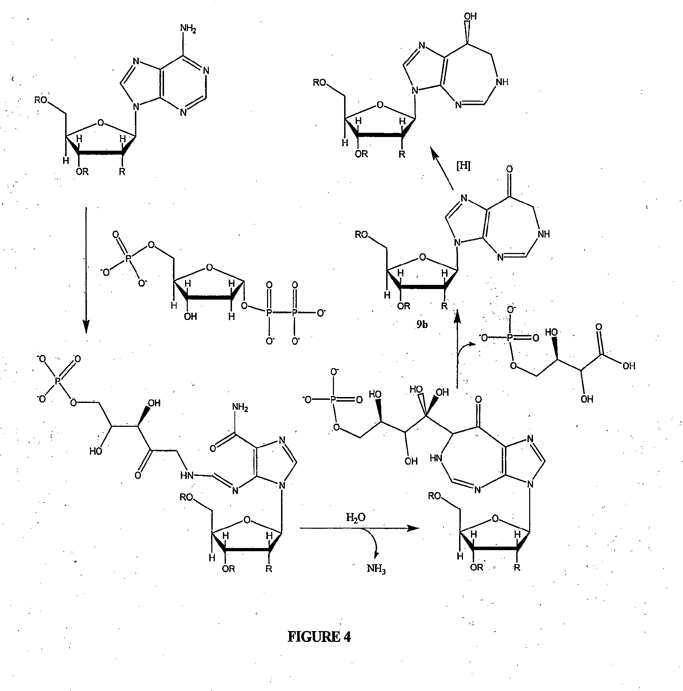

Nature, in contrast, has a very efficient pathway to synthesize pentostatin. Hanvey *et al.* has identified 8-ketocoformycin and 8-ketodeoxycoformycin **9a** as intermediates in the biosynthesis of coformycin and pentostatin by *S. antibioticus* (**Figure 4**). Hanvey, J. C.; Hawkins, E. S; Tunac, J. B.; Dechter, J. J.; Baker, D. C.; Suhadolnik, R. J. Biochemistry 1987, 26, 5636-5641; and Hanvey, J. C.; Hawkins, E.S.; Baker, D. C.; Suhadolnik, R. J. Biochemistry 1988, 27, 5790-5795. Formation of the 1,3-diazepine ring comes about by a ring expansion of the adenine moiety of adenosine with the C-1 of D-ribose. Then, reduction of the 8-keto functional group occurs stereospecificly to either coformycin or pentostatin. US 4014769 discloses a synthesis of coformycin based on ring expansion.

In view of the disadvantages associated with the different synthesis schemes of pentostatin described above, there exists a need for a high yield, efficient chemical synthesis of pentostatin, pentostatin derivatives and analogs, which does not require biosynthesis of pentostatin by microorganisms.

### SUMMARY OF THE INVENTION

Methods and compositions are provided for efficiently preparing and manufacturing pentostatin, its precursors, analogs and derivatives, and other heterocyclic compounds.

In one aspect of the invention, a method is provided for total chemical synthesis of pentostatin via a route of heterocyclic ring expansion. In one embodiment, the method comprises:
providing a hypoxanthine derivative wherein at least one of the imidazole secondary amine and the cyclic O-C-N functionality (i.e., O=C-NH or HO-C=N) is protected by a protective group;
expanding the 6-member ring of the hypoxanthine derivative to form a protected diazepinone precursor having the formula 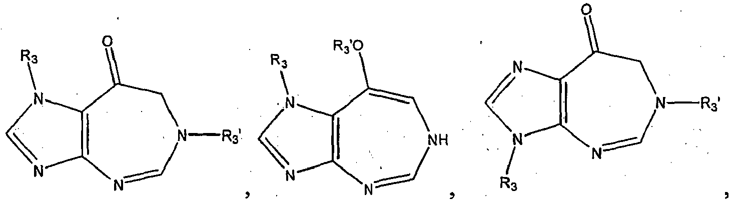 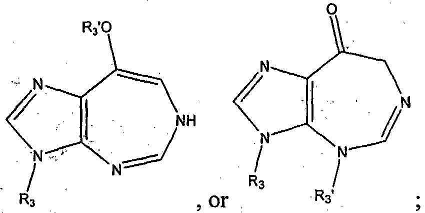
deprotecting the protected diazepinone precursor to yield a diazepinone precursor **8a** having the formula 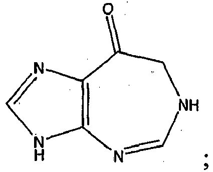
condensing the N-2 of the diazepinone precursor **8a** with the C-1 of 2-deoxy-D-ribose or its derivative to yield an intermediate having the formula **9a** 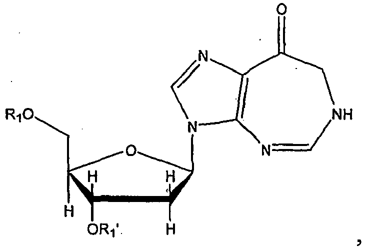 and
reducing the 8-keto functional group of compound **9a** to yield pentostatin, wherein R₁ and R₁' are each independently H or a protective group, and R₃ and R₃' are each independently H or a protective group.

Examples of protective groups R₃ and R₃' for the imidazole secondary amine and the cyclic O-C-N functionality include, but are not limited to: carbamates (e.g., methyl, ethyl, *t-*butyl, benzyl, 9-fluorenylmethyl, 2,2,2-trichloroethyl, 1-methyl-1-(4-biphenyl)ethyl, and 1-(3,5-di-*t*-butyl)-1-methylethyl); amides (e.g., acetamide, trifluoroacetamide, and benzamide); aryl amines (e.g., benzylamine, 4-methoxybenzylamine, and 2-hydroxybenzylamine); and silyl amines.

In another embodiment, the method comprises:
providing a 2'-deoxyinosine derivative wherein at least one of the hypoxanthine oxygen, the hypoxanthine amide nitrogen, the 3'-hydroxyl oxygen, and 5'-hydroxyl oxygen is protected by a protective group;
expanding the O-C-N functionality of the 6-member ring of a 2'-deoxyinosine derivative to produce an intermediate having the formula **20a** or **20b** 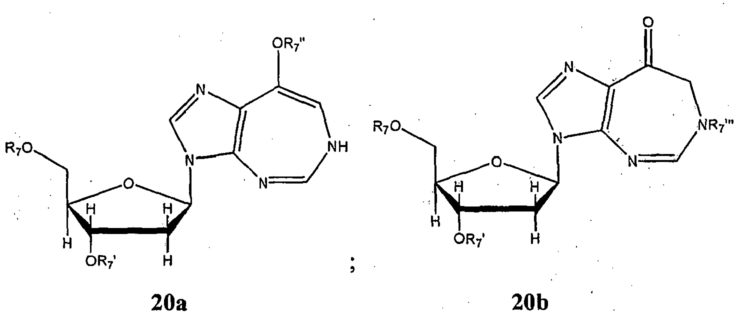 and
deprotecting and reducing the 8-keto functionality of compound **20a** or **20b** to yield pentostatin, wherein R₇, R₇', R₇" and R₇"' are each independently H or a protective group.

Examples of protective groups R₇ and R₇' include, but are not limited to benzyl ethers (e.g., *p*-methoxybenzyl, 3,4-dimethoxybenzyl, nitrobenzyl, and *p*-cyanobenzyl); silyl ethers (e.g., triakylsilyl and alkoxydialkylsilyl); esters (e.g., acetate, halogenatedacetate, alkoxyacetate, and benzoate).

Examples of protective group R₇" include, but are not limited to, benzyl ethers (e.g., *p*-methoxybenzyl, 3,4-dimethoxybenzyl, nitrobenzyl, and *p*-cyanobenzyl); and silyl ethers (e.g., triakylsilyl and alkoxydialkylsilyl).

R₇'" may be a carbamate protective group (e.g., methyl carbamate, ethyl carbamate, *t-*butyl carbamate, benzyl carbamate, 9-fluorenylmethyl carbamate, 2,2,2-trichloroethyl carbamate, 1-methyl-1-(4-biphenyl)ethyl carbamate, and 1-(3,5-di-*t*-butyl)-1-methylethyl carbamate).

According to the method, the O-C-N functionality of the 6-member ring of the 2'-deoxyinosine derivative may be expanded by reacting the 2'-deoxyinosine derivative with diazomethane or trimethylsilyldiazomethane in the presence of a Lewis acid catalyst, and preferably with anhydrous solution of diazomethane or trimethylsilyldiazomethane in ether.

Examples of the Lewis acid catalyst is include, but are not limited to, trimethylsilyl triflate (TMSOTf), BX₃, AlX₃, FeX₃, GaX₃, SbX₅, SnX₄, AsX₅, ZnX₂, and HgX₂, where X is a halogen. Preferably, the Lewis acid catalyst is ZnCl₂ or HgBr₂.

In another aspect of the invention, a method is provided for preparing coformycin. The method comprises
providing an inosine derivative wherein at least one of the hypoxanthine oxygen, the hypoxanthine amide nitrogen, the 2'-hydroxyl oxygen, the 3'-hydroxyl oxygen, and 5'-hydroxyl oxygen is protected by a protective group;
expanding the O-C-N functionality of the 6-member ring of the 2'-inosine derivative to produce an intermediate having the formula **21a** or **21b** 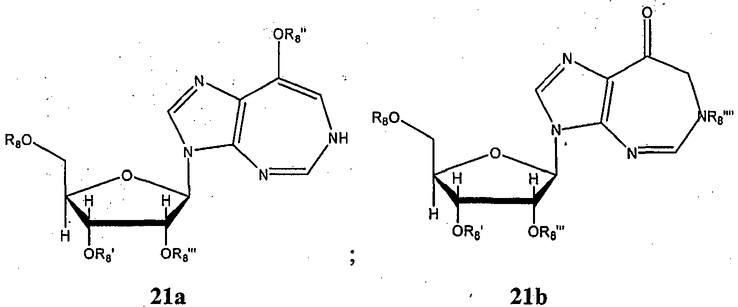 and
deprotecting and reducing the 8-keto functionality of compound **21a** or **21b** to yield coformycin, wherein R₈, R₈', Rg", R₈"' and R₈"" are each independently H or a protective group.

The R₈, R₈' and R₈'" protective groups may be benzyl ethers, silyl ethers, or esters. R₈" protective group may be a benzyl ether and silyl ether. R₈"" may be a carbamate protective group.

According to the method, the O-C-N functionality of the 6-member ring of the inosine derivative may be expanded by reacting the inosine derivative with diazomethane or trimethylsilyldiazomethane in the presence of a Lewis acid catalyst, and preferably with anhydrous solution of diazomethane or trimethylsilyldiazomethane in ether.

Examples of the Lewis acid catalyst is include, but are not limited to, trimethylsilyl triflate (TMSOTf), PX₃, AlX₃, FeX₃, GaX₃, SbX₅, SnX₄, AsX₅, ZnX₂, and HgX₂, where X is a halogen. Preferably, the Lewis acid catalyst is ZnCl₂ or HgBr₂.

In another aspect of the invention, a precursor of pentostatin or other heterocyclic compounds is provided that has the formula 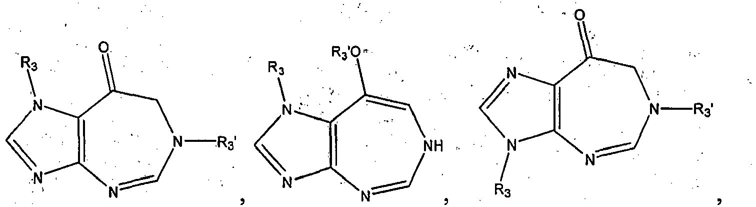 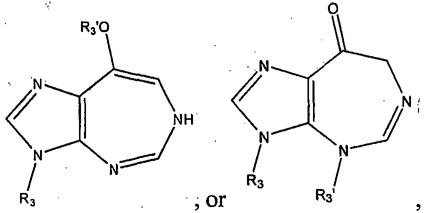 wherein R₃ and R₃' are each independently H or a protective group, and at least one of R₃ and R₃' is a protective group.

In yet another aspect of the invention, a method for manufacturing a precursor of pentostatin, diazepinone precursor **8a**, is provided. In one embodiment, the method comprises:
protecting hypoxanthine at one or more locations using a protecting group; reacting the protected hypoxanthine under a suitable condition in a appropriate solvent to yield a protected diazepinone precursor; precipitating the protected diazepinone precursor, and deprotecting the protected diazepinone precursor to yield the diazepinone precursor 8a.

In yet another aspect of the invention, a method for manufacturing pentostatin is provided. In one embodiment, the method comprises: protecting hypoxanthine at one or more locations using a protecting group; reacting the protected hypoxanthine under a suitable condition in an appropriate solvent to yield a protected diazepinone precursor; deprotecting the protected diazepinone precursor to yield the diazepinone precursor **8a;** condensing the N-2 of the diazepinone precursor **8a** with the C-1 position of 2-deoxy-D-ribose and derivatives to yield an intermediate **9a,** and deprotecting and reducing the 8-keto functional group of the intermediate **9a** to pentostatin.

In another embodiment, the method for manufacturing pentostatin comprises:
protecting 2'-deoxyinosine at one or more locations using a protecting group; expanding the 6-member ring of the protected 2'-deoxyinosine to yield an intermediate **9a;** and deprotecting and reducing the 8-keto functional group of the intermediate **9a** to yield pentostatin, wherein R₁ and R₁' are each independently a protective group.

In aspect of the invention, a method for manufacturing coformycin is provided. The method comprises:
protecting inosine at one or more locations using a protecting group;
expanding the O-C-N functionality of the 6-member ring of a protected inosine to yield an intermediate having the formula **22** 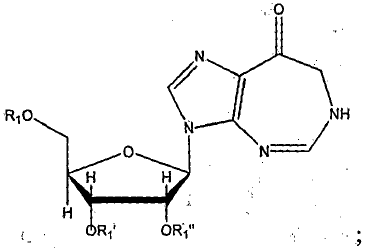 and
deprotecting and reducing the 8-keto functional group of the intermediate **22** to yield coformycin, wherein R₁, R₁' and R₁" are each independently H or a protective group.

The methods and compositions described above can also be used to synthesize and manufacture heterocyclic compounds other than pentostatin, especially pharmaceutically important heterocyclic compounds such as coformycin. Pentostatin, its precursors and derivatives may be used as therapeutic or diagnostics in the treatment of various diseases or conditions, such as hematological disorders, cancer, autoimmune diseases, and graft-versus-host disease.

### BRIEF DESCRIPTION OF THE DRAWINGS

**Figure 1A** is a scheme for synthesis of a diazepinone precursor by Showalter and Baker (Chan, E.; Putt, S. R.; Showalter, H. D. H.; Baker, D. C. J. Org. Chem. 1982, 47, 3457-3464).
**Figure 1B** is a scheme for glycosylation with peracylglycosyl chloride (Chan et al., *supra*)
**Figure 1C** is a scheme for non-stereocontrolled reduction (Chan et al., *supra*).
**Figure 2** is a scheme for synthesis of pentostatin improved by Chen (Chen, B.-C.; Chao, S. T.; Sundeen, J. E.; Tellew, J.; Ahmad, S. Tetrahedron Lett. 2002, 43, 1595-1596.).
**Figure 3** is a scheme for stereocontrolled synthesis of the Cyclopentyl analogue of pentostatin (Ho, J. Z.; Mohareb, R. M.; Ahn, J. H.; Sim, T. B.; Rapoport, H. J. Org. Chem. 2003, 68, 109-114).
**Figure 4** illustrates the mechanism and intermediates for the biosynthesis of pentostatin by *S*. *antibioticus,* shown without phosphorylation.
**Figure 5** is a scheme for introduction of R₂ protective group onto hypoxanthine, where R₂ can be any protective group.
**Figure 6A** is a scheme for ring-expansion of protected hypoxanthines to diazepinone derivatives, where R₃ can be any protective group.
**Figure 6B** shows a mass spectrum of isomeric mixture dibenzyl 6,7-dihyroimidazo[4,5-*d*][1,3]diazepin-8-(3H)-one with *m*/*z* = 331 [M + H]⁺ and a doubly-repeated ring expanded impurity at with *m*/*z* = 345 [M + H]⁺.
**Figure 6C** shows a ¹H NMR of isomeric mixture of dibenzyl 6,7-dihyroimidazo[4,5-*d*][1,3]diazepin-8-(3H)-one, with DMSO at 2.50 ppm.
**Figure 6D** shows the presence of the methylene (-CH2-) functional group of all three isomers of dibenzyl 6,7-dihyroimidazo[4,5-*d*][1,3]diazepin-8-(3H)-one at 4.12 to 3.92 ppm.
**Figure 6E** shows a 400 MHz ¹H NMR spectrum of an isomeric mixture of dibenzyl hypoxanthines in d₆-DMSO.
**Figure 6F** is an expanded view of a 400 MHz ¹H NMR spectrum of three isomeric dibenzyl hypoxanthines in d₆-DMSO between 6.0 to 2.0 ppm.
**Figure 6G** shows the dependence of the magnitude of the geminal coupling constant on the HCH angle.
**Figure 6H** lists samples of or *²J* coupling constants.
**Figure 6I** shows a 400 MHz ACD/HNMR spectrum of dibenzyl 6,7-dihyroimidazo[4 5-*d*][1,3]diazepin-8-(3H)-one in non-polar and non-aromatic solvent.
**Figure 6J** is an expanded view of a 400 MHz ACD/HNMR spectrum of dibenzyl 6,7-dihyroimidazo[4,5-*d*][1,3]diazepin-8-(3H)-one between 4.30 to 3.90 ppm, where the cyclic-CH₂- group appears, in non-polar and non-aromatic solvent.
**Figure 7** shows a scheme for deprotection of protected diazepinones to diazepinone **8a**.
**Figure 8** shows a scheme for modifications of the condensation condition to improve handling and scalability.
**Figure 9** shows a synthetic scheme for improving efficiency and yield of diazepinone condensation with 2-deoxy-D-ribose.
**Figure 10** is a scheme for improved deprotection and other asymmetric reductions of the 8-keto functional group to pentostatin.
**Figure 11** is an example of a variation on the synthesis of pentostatin by ring expansion.
**Figure 12** is another example of a variation on the synthesis of pentostatin by ring expansion.
**Figure 13** is another example of a variation on the synthesis of pentostatin by ring expansion.
**Figure 14** is an example of synthesis of a variation on the synthesis of coformycin by ring expansion.

### DETAILED DESCRIPTION OF THE PRESENT INVENTION

The present invention provides novel compositions and methods for efficiently preparing and manufacturing pentostatin. Also provided are novel precursors of pentostatin, pentostatin analogs and derivatives.

In one aspect of the invention, a method is provided for a total chemical synthesis of pentostatin via a route of heterocyclic ring expansion. Specifically, a heterocyclic pharmaceutical intermediates for drugs such as pentostatin, e.g., the diazepinone precursor **8a** and intermediate **9a**, can be obtained efficiently through a ring expansion of an O-C-N functionality in a hypoxanthine derivative or a protected 2'-deoxyinosine. The inventors believe that the ring expansion is a very efficient route for chemical transformation from a readily available, economic starting material to a complex, active pharmaceutical molecule. By using this ring expansion method, the diazepinone precursors **8a** and **9a,** which are important precursor and intermediate of pentostatin, can be obtained by no more than three synthetic steps, which tremendously reduces the total number of steps necessary to synthesize pentostatin. The diazepinone precursor **8a** can be condensed with 2-deoxy-D-ribose (or other derivatives) in various ways and intermediate **9a** reduced to yield pentostatin (or other derivatives or analogs of pentostatin).

It should be noted that the methodology and precursors provided herein can also be applied to the synthesis of compounds containing other heterocycles that require expansion of the heterocyclic ring at an O-C-N functionality, e.g., synthesis of heterocycles starting from inosine and 2'-deoxyinosine.

In some embodiments of the present invention, synthesis of pentostatin includes three parts: Module A-preparation of a diazepinone base; Module B-condensation of heterocyclic base with 2-deoxy-D-ribose; and Module C-formation of 8-(R)-hydroxyl group. According to these embodiments, there are three modular variations in the process of synthesizing pentostatin: ABC; ACB; and BAC. It should be noted that any variations based on these modules are within the scope of the present invention. The foundation of each variation is ring expansion of the O-C-N functionality of a hypoxanthine, 2'-deoxyinosine, or inosine into a cyclic α-aminoketone diazepinone. Each variation offers distinct advantages but may vary in the degrees of overall robustness, efficiency, yield and economy.

### 1. Module A-Synthesis of diazepinone precursor 8a (6,7-Dihyroimidazo[4,5-d][1,3]diazepin-8-(3H)-one)

The present invention provides an effective chemical method for synthesizing the key intermediate of pentostatin, diazepinone precursor **8a.** As outlined below, the diazepinone precursor **8a** can be obtained via a route of ring expansion of hypoxanthine.

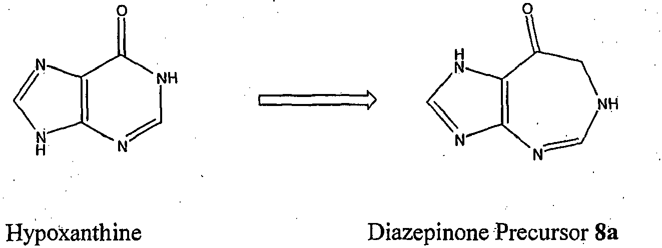

In general, the ring expansion of hypoxanthine may be achieved by direct insertion of a methylene function with a reagent such as diazomethane, which can be photocatalyzed (Doering, W. von E.; Knox, L. H. J. Am. Chem. Soc. 1951, 75, 297-303) or Lewis acid-catalyzed (Wittig, von G.; Schwarzenbach, K. Liebigs Ann. Chem. 1961, 650, 1-21). Preferably, trimethylsilyl diazomethane is used since it is a more stable reagent and may offer a safer alternative to volatile diazomethane (Seyferth, D.; Menzel, H.; Dow, A. W.; Flood, T. C. J. Organometallic Chem. 1972, 44, 279-290).

According to the present invention, diazomethane and trimethylsilyl, diazomethane are utilized to expand a heterocyclic ring containing an O-C-N functionality. The ring expansion chemistry of the present invention is applicable to all suitably protected O-C-N and cyclic O-C-N. In addition, the ring expansion can be controlled specifically and kinetically to yield only the required number of ring expansion, which is accomplished by incorporating appropriate protection groups and under a suitable solvent condition.

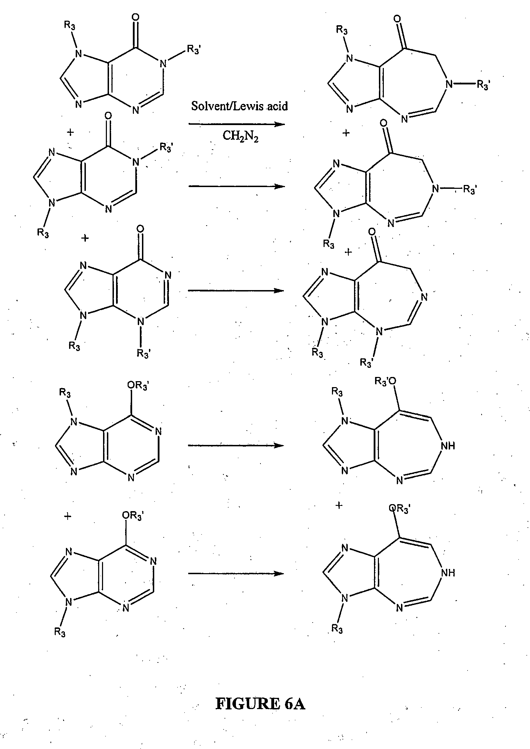

In one embodiment, the method of synthesizing diazepinone precursor **8a** involves treating a suitably protected hypoxanthine derivative, in a solution of an organic solvent with a Lewis acid catalyst and under anhydrous atmosphere, with a freshly prepared anhydrous solution of diazomethane in ether. **Figure 6A** illustrates examples of such a synthesis scheme. The reaction occurs at -78 °C to 25 °C for a period of a few minutes to a few hours.

With regard to the starting material, hypoxanthine, 2'-deoxyinosine or inosine, the heterocyclic hypoxanthine ring does not contain any stereocenter. It is commercially available from such sources as Alfa Aesar, a Johnson Matthey Co., Ward Hill, MA and Aldrich Chemical Co., Milwaukee, WI. While any purity of hypoxanthine, 2'-deoxyinosine, and inosine can be used, at least about 92% purity is preferable. The amount of hypoxanthine, 2'-deoxyinosine, or inosine used can be any amount, as long as there are sufficient amounts of the protective groups and a base effective in assisting the formation of a nucleophilic hypoxanthine ion ring to make protected hypoxanthine derivatives.

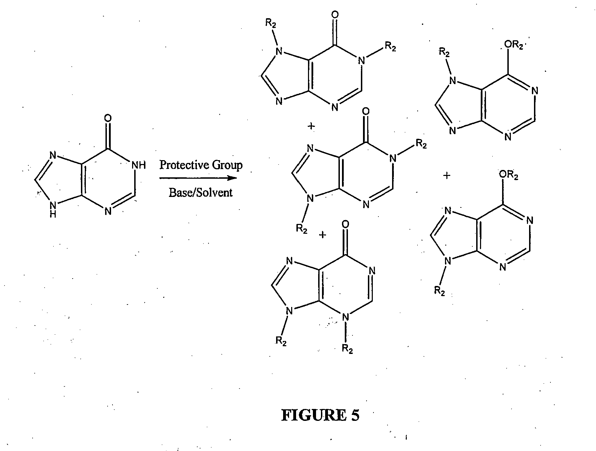

The base can be any base that is capable of forming a nucleophilic hypoxanthine ion or inducing the protective reagent in a matter that results in the protection of the imidazole secondary amine and the O-C-N functionality. The protection occurs at least on the imidazole secondary amine. Examples of bases include, but are not limited to: pyridine; aqueous NaOH; NEt₃; DMAP; K₂CO₃; Na₂CO₃; NaH; Na/NH₃; MeLi; and *t*-BuOK. **Figure 5** shows a scheme for introduction of R₂ and R₂' protective group onto hypoxanthine, where R₂ and R₂' can be any protective groups and can be the same or different from each other.

The protective groups are all groups that are capable of forming a covalent bond with all imidazole secondary amines, cyclic amine and the O-C-N (O=C-NH↔HO-C=N) functionality in the hypoxanthine ring, thereby protecting hypoxanthine ring of hypoxanthine, 2'-deoxyinosine, and inosine by any combination and variation thereof. Added features of all applicable protective groups are that they 1) help to make the hypoxanthine, 2'-deoxyinosine, and inosine derivative to be more soluble in an organic solvent, 2) allow the ring-expansion to proceed without side-reaction and decomposition (i.e. they are stable under the condition of diazomethane and a Lewis acid), and 3) assist isolation and purification of the diazepinone without interfering with the ring-expansion. Examples of protective groups for the imidazole secondary amine and the O-C-N functionality include, but are not limited to: carbamates (i.e. methyl, ethyl, *t*-butyl, benzyl, 9-fluorenylmethyl, 2,2,2-trichloroethyl, 1-methyl-1-(4-biphenyl)ethyl, and 1-(3,5-di-*t*-butyl)-1-methylethyl); amides (i.e. acetamide, trifluoroacetamide, and benzamide); aryl amines (i.e. benzylamine, 4-methoxybenzylamine, and 2-hydroxybenzylamine); and silyl amines.

With respect to the Lewis acid used for ring-expansion, the acid is effective in catalyzing the reaction specifically at the C-N bond of the O-C-N functionality, which inserts a methylene group between the O-C-N bond to form a separate ketone functionality and an amine functionality, forming the so called α-aminoketone. Examples of acids include, but are not limited to: trimethylsilyl triflate (TMSOTf); BX₃; AlX₃; FeX₃; GaX₃; SbX₅; SnX₄; AsX₅; ZnX₂; and HgX₂, where X is a halogen. The amount of Lewis acid used in the reaction should be in the range of 1% to 200% stoichiometric equivalents.

With respect to the organic solvent, the solvent is effective in solubilizing the starting material for the reaction to progress without hindrance and in a timely matter, and it should not hinder isolation and purification. Preferably, these organic solvents should be restricted to solvents acceptable for pharmaceutical processing, which include, but are not limited to: acetonitrile; chlorobenzene; dichloromethane; methylcyclohexane; N-methylpyrrolidone; nitromethane; acetone; DMSO; ethyl acetate; ethyl ether; and ethyl formate.

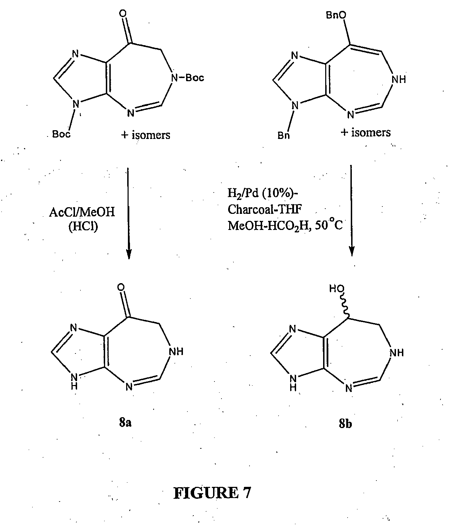

In one embodiment, the protected hypoxanthine, 2'-deoxyinosine, or inosine is added to a reaction vessel with a stirring bar for mixing before adding the selected organic solvent and Lewis acid, while maintaining anhydrous atmosphere. The stirred mixture is clear and homogeneous at -78 °C to 25 °C. While maintaining anhydrous atmosphere, enough freshly prepared diazomethane in ether is slowly added to prevent over-bubbling and addition of too much diazomethane; the mixture is continual stirred for a period of a few minutes to a few hours until the protected hypoxanthine, 2'-deoxyinosine, or inosine is completely consumed, as determined by TLC or HPLC. Once the protected hypoxanthine, 2'-deoxyinosine, or inosine is completely consumed, the protected diazepinone has already started precipitating out of the reaction mixture; an anti-solvent, preferably a solvent acceptable for pharmaceutical processing similar to those described above, is added to further precipitate the ring-expanded product, the desired diazepinone derivative. The diazepinone derivative may be purified by a suitable recrystallization solvent or SiO₂ flash column chromatography. Each diazepinone derivative is then subjected to a deprotection procedure specific to its chemistry to give diazepinone precursor **8a** and intermediate **9a** (e.g., **Figure 7**).

Two factors may be considered determining which deprotection procedure is used: yield of diazepinone and scalability. A harsh deprotection procedure would be accompanied by significant decomposition of the diazepinone, especially if it required a prolonged period of time. Under certain conditions, the yield may be excellent at microscale but poor at grams and kilograms scales.

### 2. Module B-Synthesis of Compound 9a (3-[2-Deoxy-β-D-erythro-pentofuranosyl]-6,7-dihydroimidazo[4,5-d] [1,3]diazepin-8(3H)-one)

Once the diazepinone precursor 8a has been synthesized, a synthetic scheme is provided that improves the procedure of Showalter and Baker described above in the section of "Description of Related Art", especially with improved handling, efficiency, scalability, and yield.

In the Showalter and Baker procedure, a pertrimethylsilylated diazepinone derivative was condensed to the 2-deoxy sugar via a peracylglycosyl chloride adapted from the stannic chloride catalyzed process of Vorbrüggen at the low temperature of -35 °C in the toxic solvent 1,2-dichloroethane, which is not a pharmaceutically acceptable solvent based on its high toxicity and should be avoided.

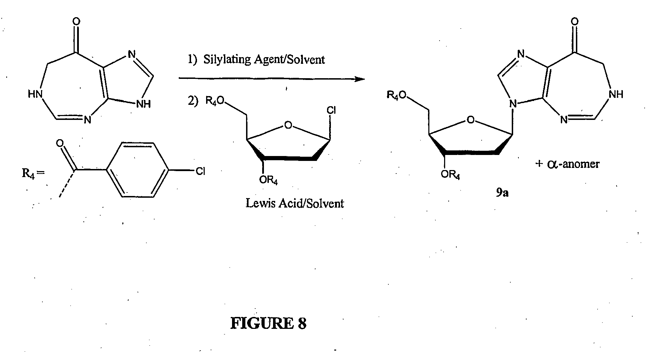

To improve this condensation, according to the present invention, weaker Lewis acids (which include, but are not limited to: ZnCl₂ and HgBr₂) may be employed. Pharmaceutically acceptable solvents (which include, but not limited to: toluene and tetrahydrofuran) that allow the condensation to occur only slowly at low temperatures should work well at elevated temperatures (0 °C to 50 °C), which should improve scalability and handling. **Figure 8** shows an embodiment of the improved condensation procedure. The α/β mixture can be separated by fractional crystallization.

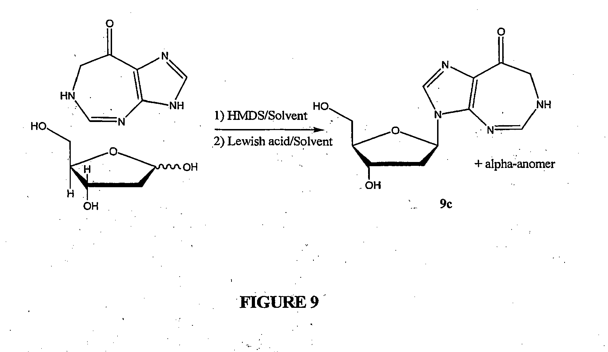

To further improve the diazepinone condensation with 2-deoxy-D-ribose, a one-pot synthesis of free nucleoside by Vorbrüggen (Bennua-Skalmowski, B.; Krolikiewicz, K.; Vorbrüggen, H. Tetrahedron Lett. 1995, 36, 7845-7848) is adapted to directly make free 6,7-dihyroimidazo[4,5-*d*][1,3]diazepin-8-(3H)-one (**Figure 9**, compound **9c**).

Persilylation of excess 2-deoxy-D-ribose and diazepinone **8a**, 2'-deoxyinosine, and inosine could be accomplished with a variety of silylating agents (which include but are not limited to: hexamethyldisilazane (HMDS); trimethylchlorosilane; bromotrimethylsilane; *N-*(trimethylsilyl)acetamide; bis-(trimethylsilyl)trifluoroacetamide; trimethylsilyl trifluoroacetate; trimethylsilyl triflate; and any combination thereof) in pharmaceutically acceptable solvents (which include, but not limited to: acetonitrile and tetrahydrofuran, respectively) for 3 hours at reflux.

Persilylation of excess 2-deoxy-D-ribose and diazepinone **8a,** and evaporation followed by condensation in presence of Lewis acid in pharmaceutically acceptable solvents (which include, but are not limited to: acetonitrile and tetrahydrofuran, respectively) with 1.1 equivalents of a Lewis acid (which include, but are not limited to: TMSOTf, BX₃, AlR₃, FeX₃, GaX₃, SbX₅, SnX₄, AsX₅, ZnX₂, and HgX₂, where X is a halogen), and transsilylation with methanolic base (which include, but not limited to: NaHCO₃ or NH₃ in methanol) should furnish free **9c,** which should be separable from the α-anomer with fractional crystallization.

In **Figure 8****,** the protection shown on 2-deoxy-D-ribose is p-toluoyl, which is just one example. Protection group R₄ includes, but are not limited to: ethers (such as methoxymethyl, benzyloxymethyl, allyl, propargyl, p-chlorophenyl, p-methoxypehenyl, p-nitrophenyl, benzyl, p-methoxybenzyl, dimethoxybenzyls, nitrobenzyl, halogenated benzyls, cyanobenzyls, trimethylsilyl, trimethylsilyl, triisopropylsilyl, tribenzylsilyl, and alkoxysilyls); esters (such as the variety of acetates and benzoates); carbonates (such as methoxymethyl, 9-fluorenylmethyl, 2,2,2-trichloroethyls, vinyl, allyl, nitrophenyls, and benzyls); sulfonates (such as allylsulfonate, mesylate, benzylsulfonate, and tosylate); cyclic acetals and ketals (such as methylene; ethylidene, acrolein, isopropylidene, cyclopentylidene, cyclohexylidene, cycloheptylidene, the variety of benzylidenes, mesitylene, 1-naphthaldehyde acetal, benzophenone ketal, o-xylyl ether); chiral ketones (such as camphor and menthone); cyclic ortho esters (such as methoxymethylene, ethoxymethylene, 1-methoxyethylidene, methylidene, phthalide, ethylidene and benzylidene derivatives, butane-2,3-bisacetal, cyclohexane-1,2-diacetal, and dispiroketals); silyl derivatives (such as di-*t*-butylsilylene and dialkylsilylene groups); cyclic carbonates; cyclic borates; and combinations and variations thereof.

### 3. Module C-Synthesis of Pentostatin ((8R)-3-(2-deoxy-β-d-erythro-pentofuranosyl)-3,6,7,8-tetrahydroimidazo [4,5-d] [1,3] diazepin-8-ol)

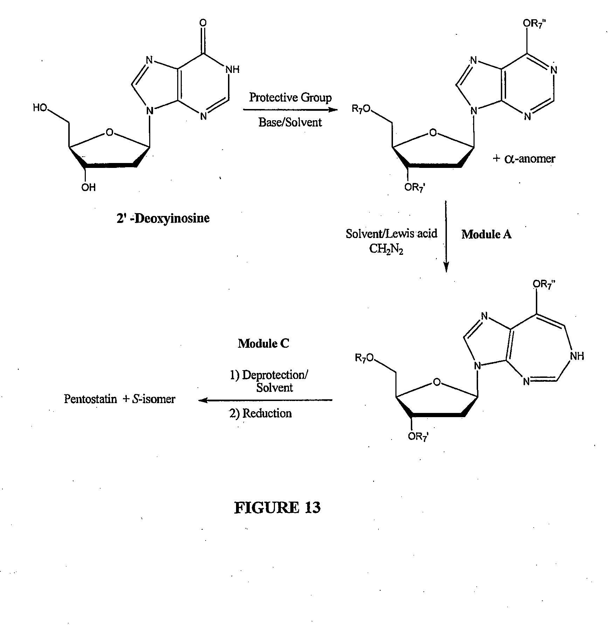

Before proceeding to reduction of the 8-keto group, if there are protective groups, they could be removed. In the Showalter and Baker procedure, methanolic sodium methoxide was used, which is a harsh environment and may lead to decomposition of the diazepinone moiety. A milder deprotection procedure (Phiasivongsa, P.; Gallagher, J.;C. Chen; P. R. Jones; Samoshin, V. V., Gross, P. H. Org. Lett. 2002, 4,4587-4590) is more preferred in order to minimize decomposition and increase the yield of free 6,7-dihyroimidazo[4,5-*d*][1,3]diazepin-8-(3H)-one (compound **9c**) before reduction **(****Figure 10**)*.* To further improve the preparation of intermediate like intermediate **9c,** the synthesis can begin with commercially available 2'-deoxyinosine as shown in **Figure 13****.**

Showalter and Baker tried a variety of sterically hindered borohydrides, but the best yielding process was determined to be the non-selective reductions with sodium borohydride and nickel catalyzed hydrogenation. Chan, E.; Putt, S. R.; Showalter, H. D. H.; Baker, D. C. J. Org. Chem. 1982, 47, 3457-3464. **Figure 10** shows a scheme for an improved method of deprotection and other asymmetric reductions of the 8-keto functional group to pentostatin.

In the present invention, hydrides doped with chiral auxiliaries are preferably implemented. Examples of hydrides include, but are not limited to, NaBH₄; LiAlH₄; BF₃-THF; and LiBH₄. Examples of chiral auxiliaries include, but are not limited to, *N,N'-*dibenzoylcystine; K glucoride; B-chlorodiisopinocampheyborane; [(1*S*)-*endo*]-(-)-borneol; and (S)-(+)- and (R.)-(-)-2-aminobutan-1-ol).

The presence of the diazepinone moiety in free 6,7-dihyroimidazo[4,5-*d*][1,3]diazepin-8-(3H)-one (**9c**) and protected derivatives like **9a** makes them good α-aminoketone candidates. This approach has been shown to work well with α-aminoketones. Asymmetric reductions of α-aminoketones with LiAH₄ treated with (S)-(+) or (R.)-(-)-2-(2-isoindolinyl)butan-1-ol (which were easily prepared in one step from commercially available (S)-(+)- and (R.)-(-)-2-aminobutan-1-ol, respectively, in high yields) to aminoalcohols with enantiomeric excess in the range of 40-97% have been achieved. Brown, E.; Leze, A; Touet, J. Tetrahedron: Asymmetry 1996, 7, 2029-2040. The inventors believe that asymmetric reductions of this type, those that have been shown to be successful for α-aminoketones, should improve the yield of pentostatin.

In addition, reduction of the 8-keto functional group may also achieved with economical hydrides (which include but are not limited to: KBH₄; NaBH₃CN; MgH₂; borohydride on Montmorillonite-KSF support; and borohydride on Amberlite® support), metals (which include but are not limited to: Li, Na or K/NH₃; Li, Na or K/alcohol; H₂ and nickel catalysts such as nickel boride and Raney nickel; H₂ and platinum catalysts; H₂ and iron catalysts such as FeCl₂; and Fe/acetic acid), and titanocene-catalyzed reduction with water and metal dust (which includes but is not limited to: zinc and manganese).

Based on the above description, a total synthesis of pentostatin may be achieved through combination of the modules A, B and C in any order. A particular synthetic pathway is modular ABC. Two other variations that are also preferred include modular ACB and BAC, which are described as follows.

### 4. Modular ACB-Total Synthesis of Pentostatin

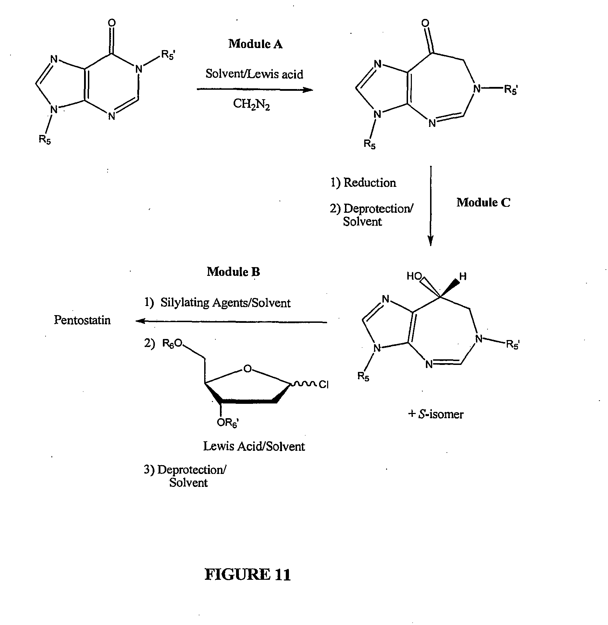

**Figure 11** shows an example of a variation on the total synthesis of pentostatin by ring expansion, where R₅ and R₅' may be any protective group and may be the same or different from each other; and R₆ and R₆' may be any protective group and may be the same or different from each other.

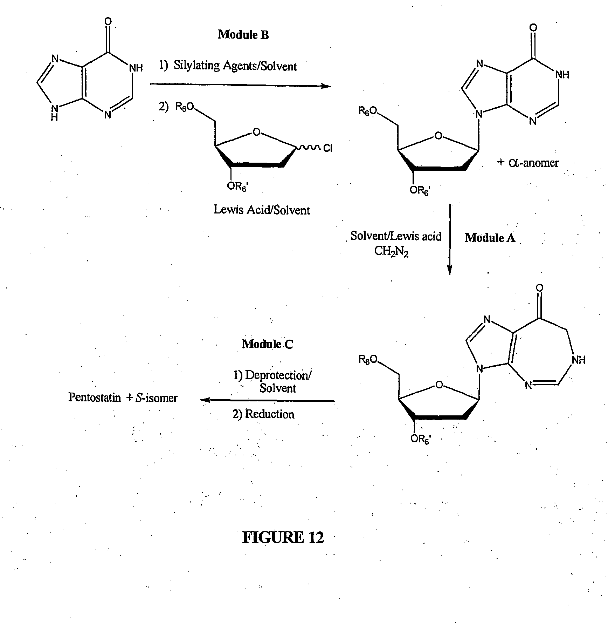

In **Figures 10** and **12**, although deprotection is performed before reduction, the reverse sequence, where the protection is left in place during reduction and then removed, could be more desirable. The different protections on the sugar (see discussion of **Figure 8**) allow the deprotection and reduction sequence to be performed in either way. In **Figure 11****,** it was sometimes desirable to perform reduction before removal of the protective groups, for reasons relating to handling, yield and purification. Then, it was necessary to remove the protective groups before condensation. After condensation, the sugar protective groups had to be removed to give the final product, pentostatin.

### 5. Modular BAC-Total Synthesis of Pentostatin

**Figure 12** shows another example of a variation on the synthesis of pentostatin by ring expansion, where R₆ and R₆' may be any protective group and may be the same or different from each other.

### 6. Modular AC-Total Synthesis of Pentostatin

Figure 13 shows another example of a variation on the synthesis of pentostatin by ring expansion, where R₇, R₇' and R₇" may be any protective group and may be the same or different from each other. Because the synthesis begins with commercially available 2'-deoxyinosine, the total synthesis of pentostatin is further shortened by bypassing at a couple of synthetic steps.

Examples of protective groups R₇ and R₇' for the hydroxyl groups in the 2-deoxyribose ring include, but are not limited to: benzyl ethers (e.g., *p*-methoxybenzyl, 3,4-dimethoxybenzyl, nitrobenzyl, and *p*-cyanobenzyl); silyl ethers (e.g., triakylsilyl and alkoxydialkylsilyl); and esters (e.g. acetate, halogenatedacetate, alkoxyacetate, and benzoate). Examples of protective group R₇" for the oxygen on the heterocyclic hypoxanthine ring include, but are not limited to: benzyl ethers (e.g., *p*-methoxybenzyl, 3,4-dimethoxybenzyl, nitrobenzyl, and *p*-cyanobenzyl) and silyl ethers (e.g., triakylsilyl and alkoxydialkylsilyl). Alternatively, the NH amide of the heterocyclic ring could be protected via transformation (NH → N-R₇"' as shown below) into carbamates (i.e. methyl, ethyl, *t*-butyl, benzyl, 9-fluorenylmethyl, 2,2,2-trichloroethyl, 1-methyl-1-(4-biphenyl)ethyl, or 1-(3,5-di-*t*-butyl)-1-methylethyl carbamate) as follows:

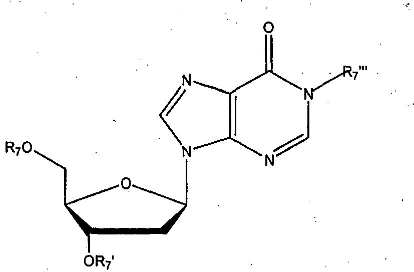

Deprotection of the benzyl ethers could be achieved with mild reagents such as the following: PhSTMS, ZnI₂, tetrabutylammonium iodide, 1,2-dichloroethane at 60 °C for 2 hours; rhodium/Al₂O₃/H₂; and Ph₃C⁺BF₄⁻ in dichloromethane. The ester protective groups could be cleaved with basic methanol and alcohols (e.g. ammonia/methanol and sodium methoxide). The silyl ethers could be easily removed with the following reagents: tetrabutylammonium fluoride in tetrahydrofuran; citric acid in methanol at 20 °C; FeCl₃ in acetonitrile at ambient temperature; and BF₃-etherate. Carbamates such as the Boc could be easily removed as described in Example 2.

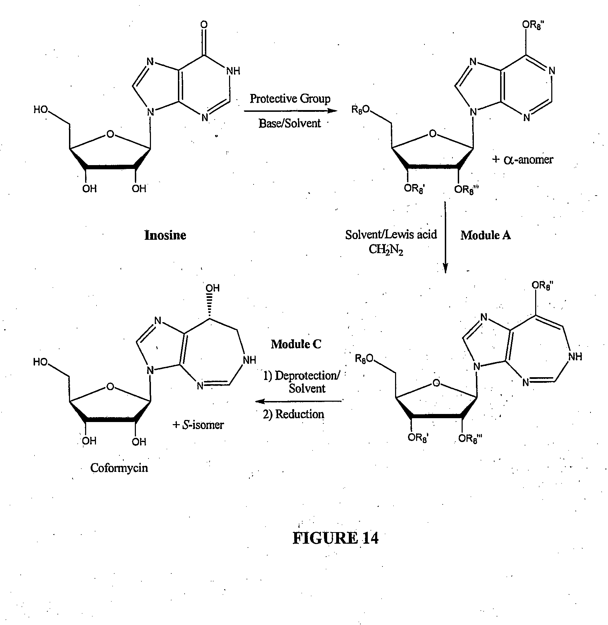

**Figure 14** shows an example of how other pentostatin derivatives, such as the synthesis of coformycin, could be achieved by ring expansion, where R₈, R₈', R₈" and R₈"' may be any protective group and may be the same or different from each other. Because the synthesis begins with commercially available inosine, the total synthesis of coformycin is further shortened by bypassing a couple of synthetic steps.

These shortened synthesis processes of pentostatin, its analogs and derivatives are highly desirable, considering the overall robustness, efficiency, yield and economy.

The following example serves to more fully describe the manner of using the above-described invention. It is understood that the example in no way serves to limit the scope of this invention, but rather is presented for illustrative purpose. All references cited herein are incorporated by reference in their entirety.

### EXAMPLES

### 1. Syntheis of Pentostatin from Dibenzyl hypoxanthine

According to the present invention, pentostatin can be synthesized through the route of ring-expansion of protected hypoxanthines to generate diazepinone derivatives as outlined in **Figure 6A** where R₃ and R₃' can be any protective group and can be the same or different from each other. In this example, pentostatin is synthesized via ring-expansion of dibenzyl-protected hypoxanthine.

To a solution containing 16 mL of water and 10g KOH in a three-neck 500 mL round bottom flask was added diethylene glycol monomethyl ether (28 mL). The flask was fitted with a simple distillation unit with a water condenser and a 250 mL receiving round bottom flask immersed in an ice-bath. A 100 mL dropping funnel-containing 10g of Diazald dissolved in ether (90 mL) was also fitted. The one unused neck of the receiver was closed with a rubber septum and balloon filled with nitrogen. The water was turned on and the distilling flask was slowly heated in an oil bath (75-80 °C) while slowly adding the Diazald solution. The rate of addition should be equal to rate of distillation, which should take about 20 min. When all the Diazald was used up, an additional 10 mL of ether was added and continue until distillate was clear. The ether should contain about 30 mmol of diazomethane.

An isomeric mixture of dibenzyl hypoxanthine (0.5 g, 1.58 mmol) was dissolved in anhydrous dichloromethane (50 mL) in a 250 mL round bottom flask before boron trifluoride diethyl etherate (0.4 mL, 2.6 mmol) was added. The mixture was closed with a rubber septum under N₂-atmosphere and cooled in an ice bath before the freshly prepared diazomethane etherate (50 mL) was slowly added via a syringe to prevent over bubbling and addition of too much diazomethane. The mixture was stirred in the ice bath for about 30 minutes under N₂-atmosphere for the reaction to be completed, as indicated by TLC (1:1:0.2 petroleum ether-ethyl acetate-methanol), at which point the product could be seen precipitating. Anhydrous diethyl ether (200 mL) was added to completely precipitate the product, and then the flask was flushed with N₂-gas, closed with a rubber septum and stored in the freezer (0 °C) overnight (12 hours). The white solid, a mixture of three isomers of dibenzyl 6,7-dihyroimidazo[4,5-*d*][1,3]diazepin-8-(3H)-one, was filtered, rinsed with diethyl ether (50 mL) and dried *in vacuo* for at least 6 hours.

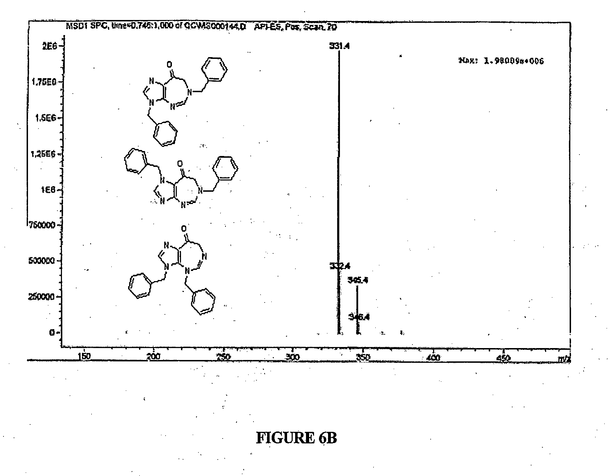

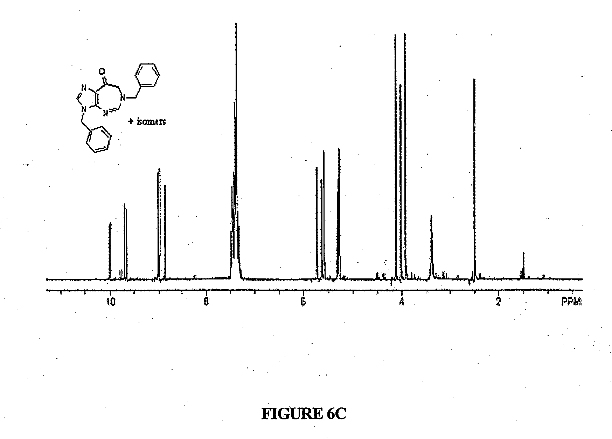

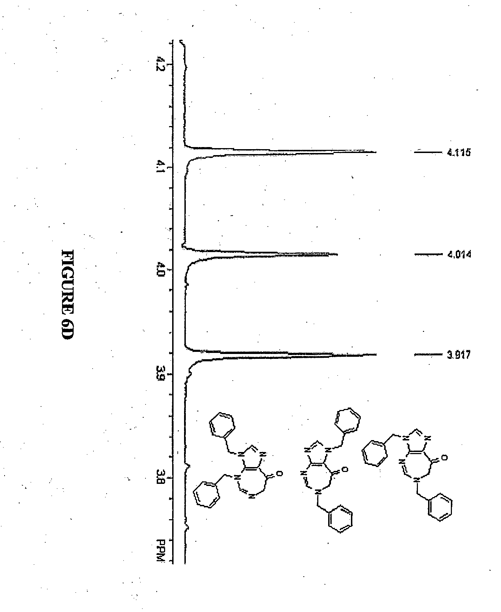

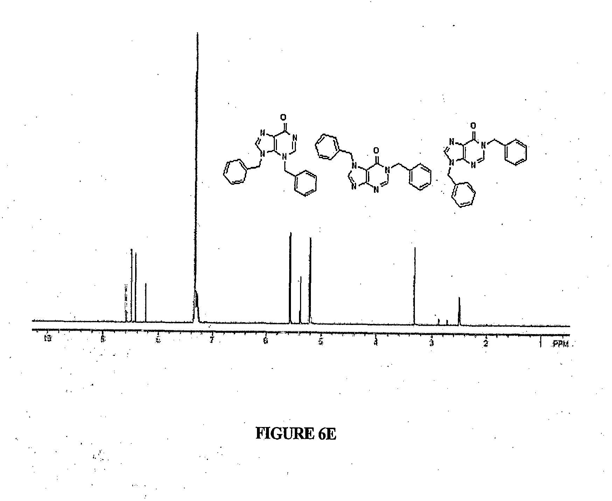

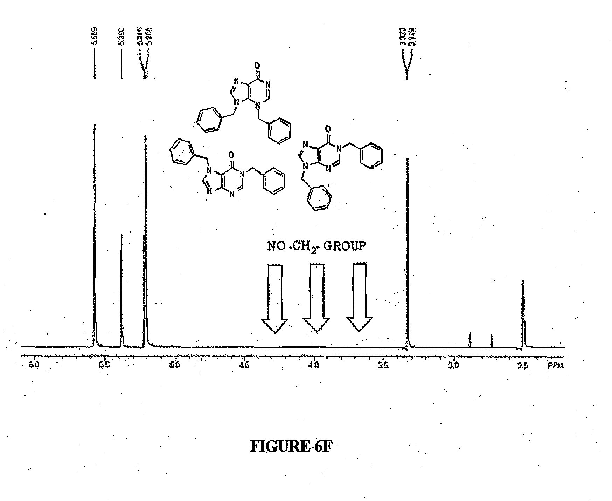

The exact mass of the three isomers of dibenzyl 6,7-dihyroimidazo[4,5-*d*][1,3]diazepin-8-(3H)-one is 330.148 Daltons compared to 316.132 Daltons for their respective dibenzyl hypoxanthine, the difference of gaining a methylene (-CH₂-) functional group. **Figures 6B** and **6C** show an API-ES mass spectrum and a 400 MHz ¹H NMR spectrum (d₆-DMSO) of the crude isomeric mixture of dibenzyl 6,7-dihyroimidazo[4,5-*d*][1,3]diazepin-8-(3H)-ones, contaminated with an 8-member ring β-aminoketone from doubly-repeated ring expansion, which gained two methylene (-CH₂-) functional groups. **Figure 6B** shows a mass spectrum of isomeric mixture dibenzyl 6,7-dihyroimidazo[4,5-*d*][1,3]diazepin-8-(3H)-one with *m*/*z* = 331 [M + H]⁺ and a doubly-repeated ring expanded impurity at with *m*/*z* = 345 [M + H]⁺. **Figure 6C** shows ¹H NMR of isomeric mixture of. dibenzyl 6,7-dihyroimidazo[4,5-*d*][1,3]diazepin-8-(3H)-one, with DMSO at 2.50 ppm. However, the β-aminoketone impurity could be removed by a variety of purification procedures (which include, but are not limited to: fractional recrystallization; column chromatography; and preparative HPLC) or prevented from forming in the first place by controlling the reaction condition to precipitate only the desired diazepinone. **Figure 6D** shows the presence of the methylene (-CH2-) functional group of all three isomers of dibenzyl 6,7-dihyroimidazo[4,5-*d*][1,3]diazepin-8-(3H)-one at 4.12 to 3.92 ppm. **Figure 6E** shows a 400 MHz ¹H NMR spectrum of an isomeric mixture of dibenzyl hypoxanthines in d₆-DMSO. **Figure 6F** shows an expanded view of a 400 MHz ¹H NMR spectrum of three isomeric dibenzyl hypoxanthines in d₆-DMSO between 6.0 to 2.0 ppm.

**Figure 6C** and the selected region of 4.2 to 3.8 ppm in **Figure 6D** clearly show the presence of the three distinct newly formed methylene (-CH₂-) singlets for the three isomers at 4.12, 4.01, and 3.92 ppm, which are obviously absent in the starting isomeric mixture of dibenzyl hypoxanthines as shown in **Figure 6E** and the selected region of 4.5 to 3.5 ppm in **Figure 6F**. The expanded view region between 6.5 and 2.0 ppm (**Figure 6F**) shows only DMSO at 2.50 ppm, solvent impurities around 3.33 pm, and benzylic hydrogen nuclei (Ph-CH₂-) from 5.57 to 5.21 ppm.

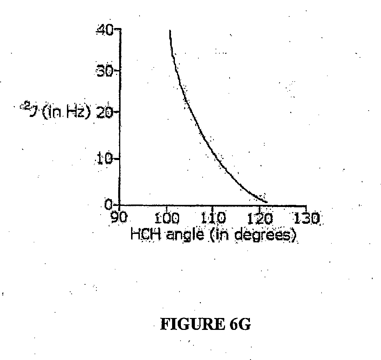

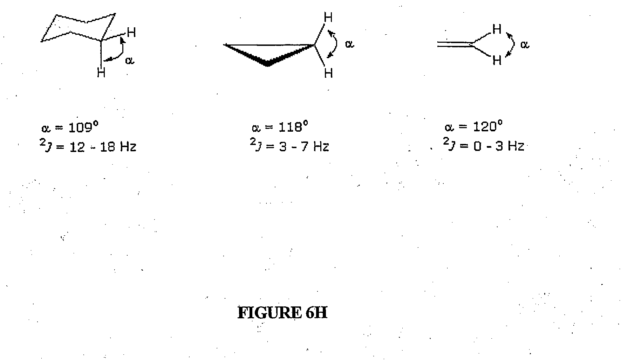

In the literature (Chan, E.; Putt, S. R.; Showalter, H. D. H.; Baker, D. C. J. Org. Chem. 1982, 47, 3457-3464), a 200 MHz ¹H NMR spectrum of unprotected diazepinone **8a** in d₆-DMSO had the methylene (-CH₂-) functional group appearing as a singlet at 4.37 ppm, which is close to those of dibenzyl 6,7-dihyroimidazo[4,5-*d*][1,3]diazepin-8-(3H)-ones. However, the splitting pattern and chemical shifts of the unprotected diazepinone **8a** may not be fully representative of dibenzyl 6,7-dihyroimidazo[4,5-*d*][1,3]diazepin-8-(3H)-ones. The methylene (-CH₂-) hydrogen nuclei are expected to be diastereomeric and should exhibit a set of double doublet peaks due to geminal coupling (also called two-bond coupling or ²*J*) since they are part of a heterocyclic ring, and therefore very likely to experience different electronic environment. Geminal coupling constants can be large, which range from +42 to -20 Hz with typical values being around 10 to 20 Hz, and dependent upon the -CH₂- bond angle (**Figures 6G** and **6H**), influence of neighboring π bonds, ring size, and orientation of electronegative β substituents.

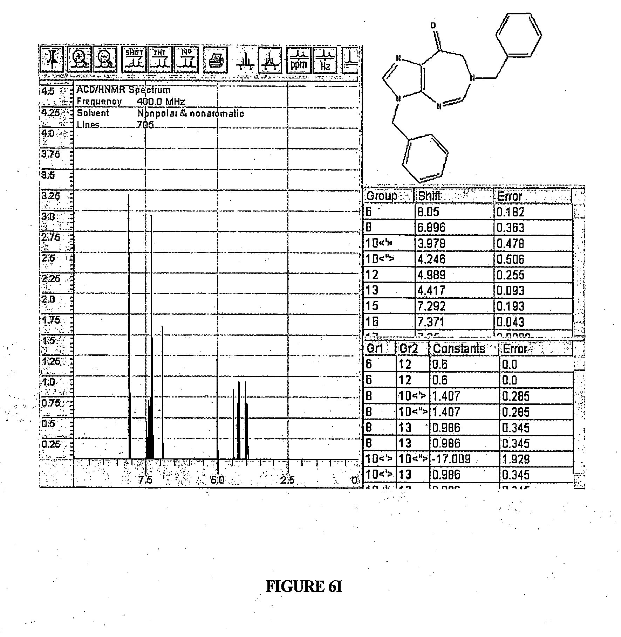

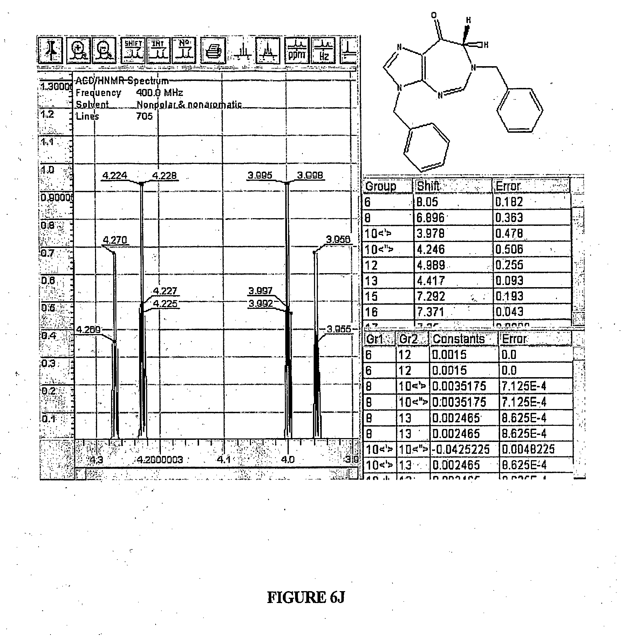

Since these protected diazepinones are novel, the ACD/I-Lab ¹H NMR Predictor, a service provided Advanced Chemistry Development, Inc. (ACD/Labs at ilab.acdlabs.com), was used to estimate splitting pattern and chemical shifts of an dibenzyl 6,7-dihyroimidazo[4,5-*d*][1,3]diazepin-8-(3H)-one (**Figures 6I** and **6J**) to further confirm the appearance of the methylene (-CH₂-) functional group. **Figure 6I** shows a 400 MHz ACD/HNMR spectrum of dibenzyl 6,7-dihyroimidazo[4,5-*d*][1,3]diazepin-8-(3H)-one in non-polar and non-aromatic solvent. **Figure 6J** shows an expanded view of a 400 MHz ACD/HNMR spectrum of dibenzyl 6,7-dihyroimidazo[4,5-*d*][1,3]diazepin-8-(3H)-one between 4.30 to 3.90 ppm, where the cyclic -CH₂- group appears, in non-polar and non-aromatic solvent.

The program predicted the methylene (-CH₂-) hydrogen nuclei to be diastereomeric, contrary to the observed data (**Figure 6D** and literature (Chan, E.; Putt, S. R.; Showalter, H. D. H.; Baker, D. C. J. Org. Chem. 1982, 47, 3457-3464)); but it was simulated in a non-polar and non-aromatic solvent and may not be entirely accurate. However, the chemical shifts for the diastereomeric hydrogen nuclei (4.25 and 3.98 ppm) were only about 0.1 ppm off from the observed methylene singlets of the three dibenzyl 6,7-dihyroimidazo[4,5-*d*][1,3]diazepin-8-(3H)-one isomers (4.12, 4.01, and 3.92 ppm). Thus, the cyclic -CH₂- nuclei of these three isomers appear to be similar and have HCH angles close to 120°.

There are numerous procedures in the literature for removal of the benzyl protective group, which include, but are not limited to: palladium-charcoal catalyzed hydrogenation with formic acid/methanol; 20% Pd(OH)₂/ethanol; Na, NH₃; *hv*, 405 nm (CuS04: NH₃); CCl₃CH₂OCOCl/acetonitrile; and RuO₄/NH₃/water. The most common is palladium-charcoal catalyzed hydrogenation with H₂ gas. For improved scalability, formamide could be used instead of H₂ gas.

As an example, dibenzyl 6,7-dihyroimidazo[4,5-*d*][1,3]diazepin-8-(3H)-one (2 mmol) is suspended in methanol (50 mL), tetrahydrofuran (25 mL) and formic acid (1.0 mL). Palladium (5%)-charcoal was added (200 mg) before the mixture is rigorously stirred under 10-30 atm of H₂ at 50 °C until complete reaction (24 to 48 hours), as indicated by TLC (1:1:0.2 petroleum ether-ethyl acetate-methanol). The catalyst is filtered over Celite and thoroughly washed with methanol. Evaporation of the filtrate leaves solid containing isomeric mixture of diazepinol **8b**.

Under dry condition, a mixture of diazepinol **8b** and *N,N*-bis(trimethylsilyl) trifluoroacetamide (6.0 mmol), pyridine (6.0 mmol), and anhydrous acetonitrile (5 mL) is stirred for not less than 12 hours or until complete reaction. Excess reagents and solvents are evaporated at 60 °C. More acetonitrile (20 mL) is added, stirred to homogeneity, and evaporated at 60 °C to give a silylated intermediate. It is re-suspended in anhydrous acetonitrile (15 mL) and cooled to -35 to -50 °C before anhydrous tin(IV) chloride (4 mmol) is added. About ten minutes later 2-deoxy-3,5-di-*O*-*p*-chlorobenzoyl-D-pentofuranosyl chloride in dry 1,2-dichloroethane is also added. The mixture is stirred for about one hour until complete reaction, as indicated by TLC (9:1 ethyl acetate-methanol). The solution is poured onto saturated bicarbonate solution (50 mL), diluted with ethyl acetate (50 mL) and filtered through Celite before the layers are separated and the aqueous layer extracted twice (2x 50 mL) with ethyl acetate. The organic layers are combined, dried with magnesium sulfate and concentrated to dryness. The desired (**8R**)- and (8S)-3-(2-deoxy-3,5-di-*O*-*p-*benzyoyl-β-D-erythro-pentofuranosyl)-3,6,7,8-tetrahydroimidazo[4,5-*d*][1,3]diazepin-8-ol are separated from the α-anomers by flash chromatography (95:5 ethyl acetate-methanol) before they are suspended in a solution of ammonia (greater than 5 fold excess) in methanol (200 mL) and the mixture is stirred at ambient temperature for 24 hours. Excess ammonia is removed, and if necessary, the methanolic solution is decolorized with activated carbon (200 mg) before evaporation to dryness. This diastereoisomeric mixture is separated by fractional crystallization in water-methanol and/or by a C-18 reverse-phase column (93:7 water-methanol) to give pure pentostatin.

### 2. Synthesis of Pentostatin from N,N-di-Boc Hypoxanthine

In this example, pentostatin is synthesized through the route of ring-expansion of *N,N-*di-Boc-protected hypoxanthines to generate diazepinone derivatives as outlined in **Figure 6A****.** **Figure 7** shows the deprotection of *N,N*-di-Boc diazepinones to diazepinone **8a** and diazepinol **8b**. Diazomethane was prepared as described in Example 1 above.

*N,N*-Di-Boc hypoxanthine (531 mg, 1.58 mmol) was dissolved in anhydrous dichloromethane (50 mL) in a 250 mL round bottom flask before boron trifluoride diethyl etherate (0.2 mL, 1.3 mmol) was added. The mixture was closed with a rubber septum under N₂-atmosphere and cooled in an ice bath before the above freshly prepared diazomethane etherate was slowly added via a syringe to prevent over bubbling and addition of too much diazomethane. The mixture was stirred in the ice bath for about 30 minutes under N₂-atmosphere for the reaction to be completed, as indicated by TLC (1:1:0.2 petroleum ether-ethyl acetate-methanol), at which point the product could be seen precipitating. Anhydrous diethyl ether or hexane (200 mL) was added, the flask was flushed with N₂-gas, closed with a rubber septum and stored in the freezer (0 °C) overnight (12 hours). The white solid *N,N*-di-*tert*-butoxycarbonyl 6,7-dihyroimidazo[4,5-*d*][1,3]diazepin-8-(3H)-one was filtered, rinsed with diethyl ether (50 mL) and dried *in vacuo* for at least 6 hours.

There are numerous procedures in the literature for removal of the Boc protective group, which include, but are not limited to: Acetyl chloride/methanol; CF₃CO₂H/PhSH; TsOH/THF/CH₂Cl₂; 10% H₂SO₄/dioxane; Me₃SiI/acetonitrile; Me₃SiCl/phenol/CH₂Cl₂; SiCl₄/phenol/CH₂Cl₂; TMSOTf/PhSCH₃; Me₃SO₃H/dioxane/CH₂Cl₂; CF₃CO₂H/ CH₂Cl₂; BF₃-Et₂O/4Å ms/CH₂Cl₂/23°C/20h; SnCl₄/AcOH/THF/CH₂Cl₂/ toluene or acetonitrile; and ZnBr₂/CH₂Cl₂. Acetyl chloride in methanol generates anhydrous HCl in methanol. This is a convenient method for removing the Boc protection to give **8a**.

Under dry condition, a mixture of diazepinone **8a**, *N*,*N*-bis(trimethylsilyl) trifluoroacetamide (6.0 mmol), pyridine (6.0 mmol), and anhydrous acetonitrile (5 mL) is stirred for not less than 12 hours or until complete reaction. Excess reagents and solvents are evaporated at 60 °C. More acetonitrile (20 mL) is added, stirred to homogeneity, and evaporated at 60 °C to give a silylated intermediate. It is re-suspended in anhydrous acetonitrile (15 mL) and cooled to -35 to -50 °C before Anhydrous tin(IV) chloride (4 mmol) is added. About ten minutes later 2-deoxy-3,5-di-*O*-*p*-chlorobenzoyl-D-pentofuranosyl, chloride in dry 1,2-dichloroethane is also added. The mixture is stirred for about one hour until complete reaction, as indicated by TLC (9:1 ethyl acetate-methanol). The solution is poured onto saturated bicarbonate solution (50 mL), diluted with ethyl acetate (50 mL) and filtered through Celite before the layers are separated and the aqueous layer extracted twice (2x 50 mL) with ethyl acetate. The organic layers are combined, dried with magnesium sulfate and concentrated to dryness. The desired 3-(2-deoxy-3,5-di-*O*-*p*-benzyoyl-β-D-*erythro*-pentofuranosyl)-3,6,7,8-tetrahydroimidazo[4,5-*d*][1,3]diazepin-8(3H)-one is separated from the α-anomer by flash chromatography (95:5 ethyl acetate-methanol) before they are suspended in a solution of ammonia (greater than 5 fold excess) in methanol (200 mL) and the mixture is stirred at ambient temperature for 24 hours. Excess ammonia is removed and solvent evaporated at 60 °C to give **9c**. The crude intermediate is dissolved in water (10 mL) and methanol (10 mL) before sodium borohydride (1 mmol) was added. The solution is stirred at ambient temperature for one hour, at the end of which excess borohydride is decomposed by addition of dry ice. Methanol is removed by evaporation, and the aqueous solution is decolorized with activated carbon (200 mg) and filtered before lyophilization to a fluffy solid. This diastereoisomeric mixture is separated by fractional crystallization in water-methanol and/or by a C-18 reverse-phase column (93:7 water-methanol) to give pure pentostatin.

### 3. Syntheis of pentostatin from 2'-deoxyinosine

In this example, pentostatin is synthesized via the route of direct ring-expansion of 2'-deoxyinosine as outlined in **Figure 13**. Diazomethane is prepared as described in Example 1.

Under dry condition, a mixture of 2'-deoxyinosine (2.0 mmol) and *N,N-*bis(trimethylsilyl) trifluoroacetamide (6.0 mmol), pyridine (6.0 mmol), and anhydrous acetonitrile (5 mL) is stirred for not less than 12 hours or until complete reaction. Excess reagents and solvents are evaporated at 60 °C. More acetonitrile (20 mL) is added, stirred to homogeneity, and evaporated at 60 °C to give per-*O*-silylated 2'-deoxyinosine. The starting material is dissolved in anhydrous dichloromethane (50 mL) in a 250 mL round bottom flask before boron trifluoride diethyl etherate (0.2 mL, 1.3 mmol) is added. The mixture is closed with a rubber septum under N₂-atmosphere and cooled in an ice bath before the above freshly prepared diazomethane etherate is slowly added via a syringe to prevent over bubbling and addition of too much diazomethane. The mixture is stirred in the ice bath for about 30 minutes under N₂-atmosphere for the reaction to be completed, as indicated by TLC. A solution of tetrabutylammonium fluoride (3.0 mmol) dissolved in tetrahydrofuran (100 mL) is slowly added, and the mixture is stirred in the ice bath for not less than two hours, at which point intermediate product **9c** can be seen precipitating. The crude intermediate is filtered, dried *in vacuo*, and then dissolved in water (10 mL) and methanol (10 mL) before sodium borohydride (1 mmol) is added. The solution is stirred at ambient temperature for one hour, at the end of which excess borohydride is decomposed by addition of dry ice. Methanol is removed by evaporation, and the aqueous solution is decolorized with activated carbon (200 mg) and filtered before lyophilization to a fluffy solid. This diastereoisomeric mixture is separated by fractional crystallization in water-methanol and/or by a C-18 reverse-phase column (93:7 water-methanol) to give pure pentostatin.

## Claims

1. A method for preparing pentostatin, comprising:
providing a hypoxanthine derivative wherein at least one of the imidazole secondary amine and the O-C-N functionality (O=C-NH or HO-C=N) is protected by a protective group;
expanding the 6-member ring of the hypoxanthine derivative to form a protected diazepinone precursor having the formula 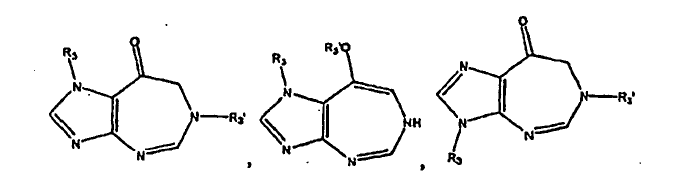 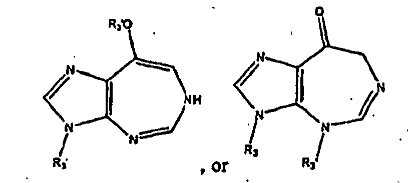
deprotecting the protected diazepinone precursor to yield a diazepinone precursor 8a having the formula 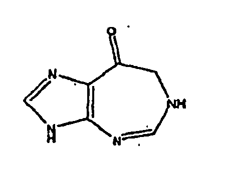
condensing the N-2 of the diazepinone precursor 8a with the C-l of 2-deoxy-D-ribose or its derivative to yield an intermediate having the formula 9a 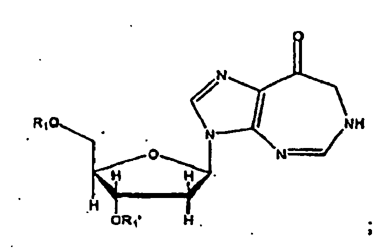 **and**
reducing the 8-keto functional group of the compound 9a to yield pentostatin,
wherein R₁ and R₁, are each independently H or a protective group, and R₃ and R_{3'} are each independently H or a protective group.

2. The method according to claim 1, wherein R₃ and R_{3'} are each independently a carbamate, amide, aryl amine or silyl amine protective group.

3. The method according to claim 2, wherein the amide protective group is selected from the group consisting of acetamide, trifluoroacetamide, and benzamide.

4. The method according to claim 2, wherein the aryl amine protective group is selected from the group consisting of benzylamine, 4-methoxybenzylamine, and 2-hydroxybenzylamine.

5. The method according to claim 1, wherein R₁ and R_{1'} are each independently a protective group selected from the group consisting of ether, ester, carbonate, sulfonate, cyclic acetal and ketal, chiral ketone, cyclic ortho ester, silyl derivative, cyclic carbonate, and cyclic borate.

6. The method according to claim 1, wherein R₁ and R_{1'} are each independently a protective group of p-toluoyl.

7. The method according to claim 5, wherein the ether protective group is selected from the group consisting of methoxymethyl, benzyloxymethyl, allyl, propargyl, p-chlorophenyl, p-methoxypehenyl, p-nitrophenyl, benzyl, p-methoxybenzyl, dimethoxybenzyls, nitrobenzyl, halogenated benzyls, cyanobenzyls, trimethylsilyl, trimethylsilyl, triisopropylsilyl, tribenzylsilyl, and alkoxysilyls.

8. The method according to claim 5, wherein the ester protective group is selected from the group consisting of acetates and benzoates.

9. The method according to claim 5, wherein the carbonate protective group is selected from the group consisting of methoxymethyl, 9-fluorenylmethyl, 2,2,2-trichloroethyls, vinyl, allyl, nitrophenyls, and benzyls.

10. The method according to claim 5, wherein the sulfonate protective group is selected from the group consisting of allylsulfonate, mesylate, benzylsulfonate, and tosylate.

11. The method according to claim 5, wherein the cyclic acetal or ketal protective group is selected from the group consisting of methylene, ethylidene, acrolein, isopropylidene, cyclopentylidene, cyclohexylidene, cycloheptylidene, benzylidenes, mesitylene, 1naphthaldehyde acetal, benzophenone ketal, and o-xylyl ether.

12. The method according to claim 5, wherein the chiral ketone protective group is selected from the group consisting of camphor and menthone.

13. The method according to claim 5, wherein the cyclic ortho ester protective group is selected from the group consisting of methoxymethylene, ethoxymethylene, 1- methoxyethylidene, methylidene, phthalide, ethylidene and benzylidene derivatives, butane-2,3-bisacetal, cyclohexane-1,2-diacetal, and dispiroketals.

14. The method according to claim 5, wherein the silyl derivative protective group is selected from the group consisting of di-t-butylsilylene and dialkylsilylene groups.

15. The method according to claim 1, wherein expanding the 6-member ring of the hypoxanthine derivative includes reacting the hypoxanthine derivative with diazomethane or trimethylsilyldiazomethane in the presence of a Lewis acid catalyst.

16. The method according to claim 15, wherein reacting the hypoxanthine derivative with diazomethane or trimethylsilyldiazomethane includes reacting the hypoxanthine derivative with anhydrous solution of diazomethane or trimethylsilyldiazomethane in ether.

17. A compound that is a precursor of pentostatin or other heterocyclic compounds having the formula 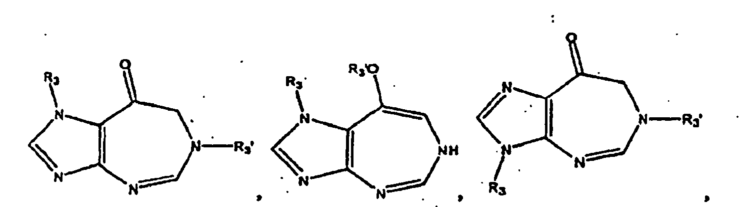 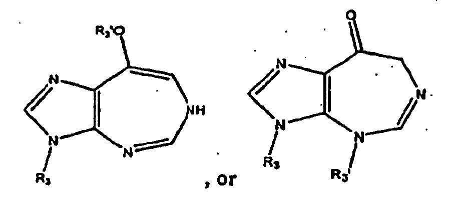 wherein R₃ and R_{3'} are each independently H or a protective group, and at least one of R₃ and R_{3'} is a protective group,
wherein the protective group is selected from a carbamate protective group, an amide protective group, an aryl amine protective group or a silyl amine protective group,
wherein the carbamate protective group is selected from the group consisting of methyl carbamate, ethyl carbamate, t-butyl carbamate, benzyl carbamate, 9-fluorenylmethyl carbamate, 2,2,2-trichloroethyl carbamate, 1-methyl-1-(4-biphenyl) ethyl carbamate, and 1-(3,5-di-t-butyl)-1-methylethyl carbamate; wherein the amide protective group is selected from the group consisting of acetamide, trifluoroacetamide, and benzamide; wherein the aryl amine protective group is selected from the group consisting of benzylamine, 4-methoxybenzylamine, and 2-hydroxybenzylamine; and
wherein the silyl protecting group is selected from the group consisting of di-t-butylsilylene, dialkylsilylene, trialkylsilyl selected from trimethylsilyl, triethylsilyl, triisopropylsilyl, dimethylisopropylsilyl, diethylisopropylsilyl, t-butyldimethylsilyl, tribenzylsilyl, triphenylsilyl, di-t-butylmethylsilyl and tris(trimethylsilyl)silyl, and alkoxydialkylsilyl selected from t-butylmethoxyphenylsilyl and t-butoxydiphenylsilyl.

18. A method for manufacturing a diazepinone precursor of pentostatin, comprising
protecting hypoxanthine at one or more locations using a protecting group;
reacting the protected hypoxanthine in an organic solvent to yield a protected diazepinone precursor having the formula 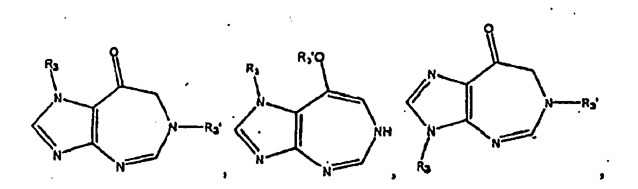 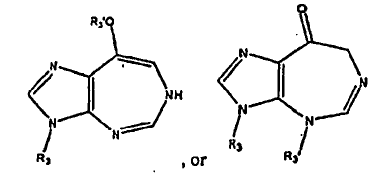 wherein R₃ and R_{3'} are each independently H or a protective group; precipitating the protected diazepinone precursor, and
deprotecting the protected diazepinone precursor to yield the diazepinone precursor 8a having the formula

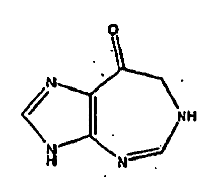

19. A method for manufacturing pentostatin, comprising:
protecting hypoxanthine at one or more locations using a protecting group;
reacting the protected hypoxanthine in an organic solvent to yield a protected diazepinone precursor having the formula 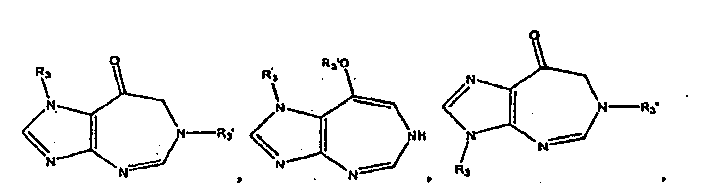 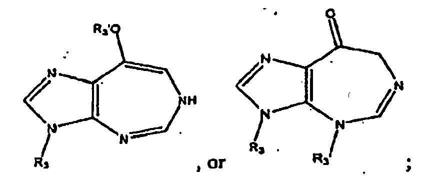
deprotecting the protected diazepinone precursor to yield the diazepinone precursor 8a having the formula 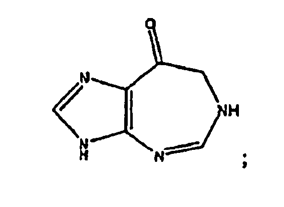
condensing the N-2 of the diazepinone precursor 8a with the C-l position of 2-deoxy-D-ribose or its derivative to yield an intermediate 9a having the formula 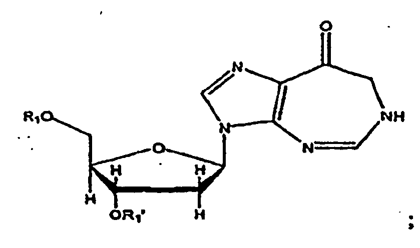 **and**
reducing the 8-keto functional group of the compound 9a to yield pentostatin, wherein R₁ and R_{1'} are each independently H or a protective group, and R₃ and R_{3'} are each independently H or a protective group.

20. The method of claims 18 or 19, wherein the organic solvent is selected from acetonitrile, chlorobenzene, dichloromethane, methylcyclohexane, N-methylpyrrolidone, nitromethane, acetone, DMSO, ethyl acetate, ethyl ether and ethyl formate.

21. The method of claims 18 or 19, wherein the protected hypoxanthine is reacted in the presence of a Lewis acid catalyst.

22. The method of claim 21, wherein the Lewis acid catalyst is selected from trimethylsilyl triflate, BX₃, AlX₃, FeX₃, GaX₃, SbX₅, SnX₄, AsX₅, ZnX₂, and HgX₂, wherein X is a halogen.

23. The method of claims 18 or 19, wherein the protected hypoxanthine is reacted under anhydrous conditions.

24. A method for preparing pentostatin, comprising:
providing a 2'-deoxyinosine derivative wherein at least one of the hypoxanthine oxygen, the hypoxanthine amide nitrogen, the 3'-hydroxyl oxygen, and 5'-hydroxyl oxygen is protected by a protective group;
expanding the O-C-N functionality (O=C-NH or HO-C=N) of the 6-member ring of the 2'-deoxyinosine derivative to produce an intermediate having the formula 20a or 20b 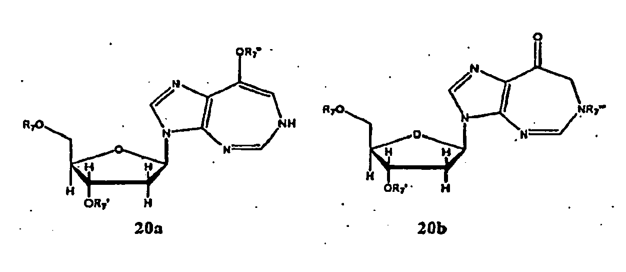 and
deprotecting and reducing the 8-keto functional group of compound 20a or 20b to yield pentostatin, wherein R₇, R_{7'}, R_{7''} and R_{7'''} are each independently H or a protective group.

25. The method according to claim 24, wherein R₇ and R_{7'} are each independently a protective group selected from the group consisting of benzyl ethers, silyl ethers, and esters.

26. The method according to claim 24, wherein R_{7''} is a protective group is selected from the group consisting of benzyl ethers and silyl ethers.

27. The method according to claim 24, wherein R_{7'''} is a carbamate protective group.

28. The method according to claim 24, wherein expanding the O-C-N functionality (O=CNH or HO-C=N) of the 6-member ring of the 2'-deoxyinosine derivative includes reacting the 2'-deoxyinosine derivative with diazomethane or trimethylsilyldiazomethane in the presence of a Lewis acid catalyst.

29. The method according to claim 27, wherein reacting the 2'-deoxyinosine derivative with diazomethane or trimethylsilyldiazomethane includes reacting the 2'-deoxyinosine derivative with anhydrous solution of diazomethane or trimethylsilyldiazomethane in ether.

30. The method according to claim 24, wherein R₇, R_{7'}, R_{7''} and R_{7"'} are each independently a silyl ether protective group.

31. The method according to claim 24, wherein deprotecting and reducing the 8-keto functional group of compound 20a or 20b includes
deprotecting compound 20a or 20b to produce a pentostatin precursor having the following formula 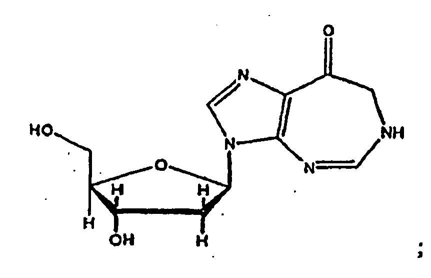 **and**
reducing the 8-keto functional group of the pentostatin precursor to yield pentostatin.

32. A method for preparing coformycin, comprising:
providing an inosine derivative wherein at least one of the hypoxanthine oxygen, the hypoxanthine amide nitrogen, the 2'-hydroxyl oxygen, the 3'-hydroxyl oxygen, and 5'-hydroxyl oxygen is protected by a protective group;
expanding the O-C-N functionality (O=C-NH or, HO-C=N) of the 6-member ring of the inosine derivative to produce an intermediate having the formula 21a or 21b 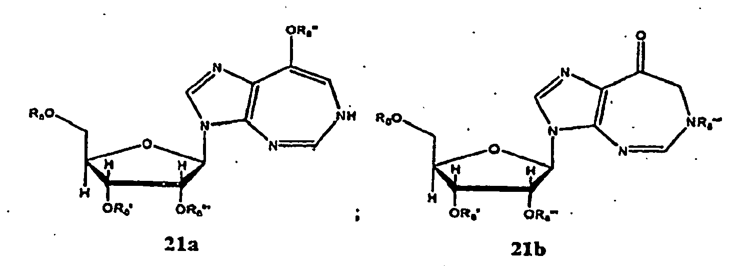 and
deprotecting and reducing the 8-keto functional group of compound 21a or 21b to yield coformycin, wherein R₈, R_{8'}, R_{8"}, R_{8"'} and R_{8""} are each independently H or a protective group.

33. The method according to claim 32, wherein R₈, R_{8'} and R_{8"'} are each independently a protective group selected from the group consisting of benzyl ethers, silyl ethers, and esters.

34. The method according to claim 33, wherein the benzyl ether protective group is selected from the group consisting of p-methoxybenzyl, 3,4-dimethoxybenzyl, nitrobenzyl, and p-cyanobenzyl.

35. The method according to any of claims 25 and 33, wherein the ester protective group is selected from the group consisting of acetate, halogenatedacetate, alkoxyacetate, and benzoate.

36. The method according to claim 32, wherein R_{8"} is a protective group is selected from the group consisting of benzyl ethers and silyl ethers.

37. The method according to any of claims 25, 26 and 36, wherein the benzyl ether protective group is selected from the group consisting of p-methoxybenzyl, 3,4-dimethoxybenzyl, nitrobenzyl, and p-cyanobenzyl.

38. The method according to any of claims 25, 26, 33 and 36, wherein the silyl ether protective group is selected from the group consisting of trialkylsilyl and alkoxydialkylsilyl.

39. The method according to claim 38, wherein the trialkylsilyl protective group is selected from the group consisting of trimethylsilyl, triethylsilyl, triisopropylsilyl, dimethylisopropylsilyl, diethylisopropylsilyl, t-butyldimethylsilyl, tribenzylsilyl, triphenylsilyl, di-t-butylmethylsilyl, and tris (trimethylsilyl) silyl.

40. The method according to claim 38, wherein the alkoxydialkylsilyl protective group is selected from the group consisting of t-butylmethoxyphenylsilyl and t-butoxydiphenylsilyl.

41. The method according to claim 32, wherein R_{e""} is a carbamate protective group.

42. The method according to any of claims 2, 27 and 41, wherein the carbamate protective group is selected from the group consisting of methyl carbamate, ethyl carbamate, t-butyl carbamate, benzyl carbamate, 9-fluorenylmethyl carbamate, 2,2,2-trichloroethyl carbamate, 1-methyl-1-(4-biphenyl) ethyl carbamate, and 1-(3,5-di-t-butyl)-1-methylethyl carbamate.

43. The method according to claim 32, wherein expanding the 6-member ring of the inosine derivative includes reacting the inosine derivative with diazomethane or trimethylsilyldiazomethane in the presence of a Lewis acid catalyst.

44. The method according to any of claims 15, 28 and 43, wherein the Lewis acid catalyst is selected from the group consisting of trimethylsilyl triflate (TMSOTf), BX₃, AIX₃, FeX₃, GaX₃, SbX₅, SnX₄, AsX₅, ZnX₂, and HgX₂, wherein X is a halogen.

45. The method according to any of claims 15, 28 and 43, wherein the Lewis acid catalyst is BF₃-Et₂O, ZnCl₂ or HgBr₂.

46. The method according to claim 43, wherein reacting the inosine derivative with diazomethane or trimethylsilyldiazomethane includes reacting the hypoxanthine derivative with anhydrous solution of diazomethane or trimethylsilyldiazomethane in ether.

## Patentansprüche

1. Ein Verfahren zur Herstellung von Pentostatin, das folgendes umfasst:
Bereitstellen eines Hypoxanthinderivats, worin mindestens eines von dem Imidazol-sekundären Amin und der O-C-N Funktionalität (O=C-NH oder HO-C=N) durch eine Schutzgruppe geschützt ist;
Erweitern des 6-gliedrigen Rings des Hypoxanthinderivats, um einen geschützten Diazepinon-Vorläufer mit der folgenden Formel zu bilden 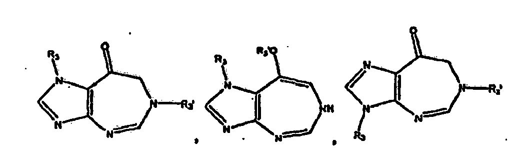 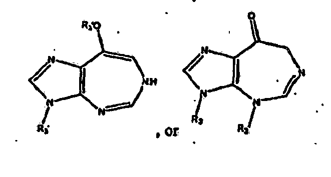
Entschützen des geschützten Diazepinon-Vorläufers, um einen Diazepinon-Vorläufer 8a mit der folgenden Formel zu ergeben 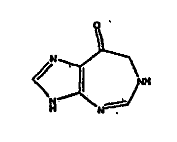
Kondensieren des N-2 des Diazepinon-Vorläufers 8a mit dem C-1 von 2-Desoxy-D-Ribose oder ihrem Derivat, um ein Intermediat mit der Formel 9a zu ergeben 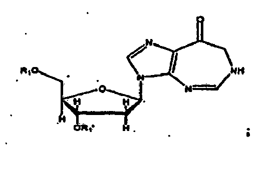 und
Reduzieren der 8-Keto funktionellen Gruppe der Verbindung 9a, um Pentostatin zu ergeben,
worin R₁ und R_{1'} jeweils unabhängig H oder eine Schutzgruppe sind, und R₃ und R_{3'} sind jeweils unabhängig H oder eine Schutzgruppe.

2. Das Verfahren gemäß Anspruch 1, worin R₃ und R_{3'} jeweils unabhängig eine Carbamat-, Amid-, Arylamin- oder Silylamin-Schutzgruppe sind.

3. Das Verfahren gemäß Anspruch 2, worin die Amid-Schutzgruppe gewählt ist aus der Gruppe bestehend aus Acetamid, Trifluoracetamid und Benzamid.

4. Das Verfahren gemäß Anspruch 2, worin die Arylamin-Schutzgruppe gewählt ist aus der Gruppe bestehend aus Benzylamin, 4-Methoxybenzylamin und 2-Hydroxybenzylamin.

5. Das Verfahren gemäß Anspruch 1, worin R₁ und R_{1'} jeweils unabhängig eine Schutzgruppe sind, gewählt aus der Gruppe bestehend aus Ether, Ester, Carbonat, Sulfonat, zyklischem Acetal und Ketal, chiralem Keton, zyklischem Orthoester, Silylderivat, zyklischem Carbonat und zyklischem Borat.

6. Das Verfahren gemäß Anspruch 1, worin R₁ und R_{1'} jeweils unabhängig eine Schutzgruppe von p-Toluoyl sind.

7. Das Verfahren gemäß Anspruch 5, worin die Ether-Schutzgruppe gewählt ist aus der Gruppe bestehend aus Methoxymethyl, Benzyloxymethyl, Allyl, Propargyl, p-Chlorphenyl, p-Methoxyphenyl, p-Nitrophenyl, Benzyl, p-Methoxybenzyl, Dimethoxybenzylen, Nitrobenzyl, halogenierten Benzylen, Cyanobenzylen, Trimethylsilyl, Trimethylsilyl, Triisopropylsilyl, Tribenzylsilyl und Alkoxysilylen.

8. Das Verfahren gemäß Anspruch 5, worin die Ester-Schutzgruppe gewählt ist aus der Gruppe bestehend aus Acetaten und Benzoaten.

9. Das Verfahren gemäß Anspruch 5, worin die Carbonat-Schutzgruppe gewählt ist aus der Gruppe bestehend aus Methoxymethyl, 9-Fluorenylmethyl, 2,2,2-Trichlorethylen, Vinyl, Allyl, Nitrophenylen und Benzylen.

10. Das Verfahren gemäß Anspruch 5, worin die Sulfonat-Schutzgruppe gewählt ist aus der Gruppe bestehend aus Allylsulfonat, Mesylat, Benzylsulfonat und Tosylat.

11. Das Verfahren gemäß Anspruch 5, worin die zyklische Acetal- oder Ketal-Schutzgruppe gewählt ist aus der Gruppe bestehend aus Methylen, Ethyliden, Acrolein, Isopropyliden, Cyclopentyliden, Cyclohexyliden, Cycloheptyliden, Benzylidenen, Mesitylen, 1-Naphthaldehydacetal, Benzophenonketal und o-Xylylether.

12. Das Verfahren gemäß Anspruch 5, worin die chirale Keton-Schutzgruppe gewählt ist aus der Gruppe bestehend aus Campher und Menthon.

13. Das Verfahren gemäß Anspruch 5, worin die zyklische Orthoester-Schutzgruppe gewählt ist aus der Gruppe bestehend aus Methoxymethylen, Ethoxymethylen, 1-Methoxyethyliden, Methyliden, Phthalid, Ethyliden und Benzylidenderivaten, Butan-2,3-bisacetal, Cyclohexan-1,2-diacetal und Dispiroketalen.

14. Das Verfahren gemäß Anspruch 5, worin die Silylderivat-Schutzgruppe gewählt ist aus der Gruppe bestehend aus di-t-Butylsilylen- und Dialkylsilylengruppen.

15. Das Verfahren gemäß Anspruch 1, worin das Erweitern des 6-gliedrigen Rings des Hypoxanthinderivats das Reagieren des Hypoxanthinderivats mit Diazomethan oder Trimethylsilyldiazomethan in der Anwesenheit eines Lewis-Säure-Katalysators einschließt.

16. Das Verfahren gemäß Anspruch 15, worin das Reagieren des Hypoxanthinderivats mit Diazomethan oder Trimethylsilyldiazomethan das Reagieren des Hypoxanthinderivats mit wasserfreier Lösung von Diazomethan oder Trimethylsilyldiazomethan in Ether einschließt.

17. Eine Verbindung, die ein Vorläufer von Pentostatin oder anderen heterozyklischen Verbindungen ist mit der folgenden Formel 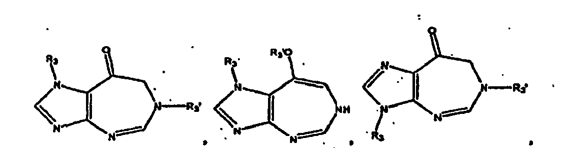 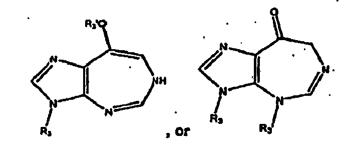 worin R₃ und R_{3'} jeweils unabhängig H oder eine Schutzgruppe sind, und mindestens eines von R₃ und R_{3'} ist eine Schutzgruppe,
worin die Schutzgruppe gewählt ist aus einer Carbamat-Schutzgruppe, einer Amid-Schutzgruppe, einer Arylamin-Schutzgruppe oder einer Silylamin-Schutzgruppe,
worin die Carbamat-Schutzgruppe gewählt ist aus der Gruppe bestehend aus Methylcarbamat, Ethylcarbamat, t-Butylcarbamat, Benzylcarbamat, 9-Fluorenylmethylcarbamat, 2,2,2-Trichlorethylcarbamat, 1-Methyl-1-(4-biphenyl) Ethylcarbamat, und 1-(3,5-di-t-butyl)-1-methylethylcarbamat;
worin die Amid-Schutzgruppe gewählt ist aus der Gruppe bestehend aus Acetamid, Trifluoracetamid, und Benzamid;
worin die Arylamin-Schutzgruppe gewählt ist aus der Gruppe bestehend aus Benzylamin, 4-Methoxybenzylamin und 2-Hydroxybenzylamin; und
worin die Silyl-Schutzgruppe gewählt ist aus der Gruppe bestehend aus di-t-Butylsilylen, Dialkylsilylen, Trialkylsilyl, gewählt aus Trimethylsilyl, Triethylsilyl, Triisopropylsilyl, Dimethylisopropylsilyl, Diethylisopropylsilyl, t-Butyldimethylsilyl, Tribenzylsilyl, Triphenylsilyl, di-t-Butylmethylsilyl und tris(Trimethylsilyl)silyl, und Alkoxydialkylsilyl gewählt aus t-Butylmethoxyphenylsilyl und t-Butoxydiphenylsilyl.

18. Ein Verfahren zur Herstellung eines Diazepinon-Vorläufers von Pentostatin, umfassend das Schützen von Hypoxanthin an einer oder mehreren Stellen unter Verwendung einer Schutzgruppe;
Reagieren des geschützten Hypoxanthins in einem organischen Lösungsmittel, um einen geschützten Diazepinon-Vorläufer mit der folgenden Formel zu ergeben 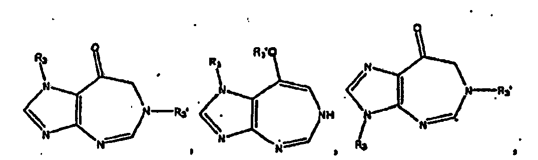 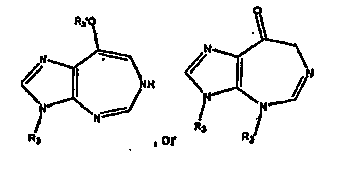 worin R₃ und R_{3'} jeweils unabhängig H oder eine Schutzgruppe sind; Ausfällen des geschützten Diazepinon-Vorläufers, und Entschützen des geschützten Diazepinon-Vorläufers, um den Diazepinon-Vorläufer 8a mit der folgenden Formel zu ergeben

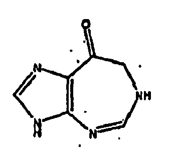

19. Ein Verfahren zur Herstellung von Pentostatin, das folgendes umfasst:
Schützen von Hypoxanthin an einer oder mehreren Stellen unter Verwendung einer Schutzgruppe;
Reagieren des geschützten Hypoxanthins in einem organischen Lösungsmittel, um einen geschützten Diazepinon-Vorläufer mit der folgenden Formel zu ergeben 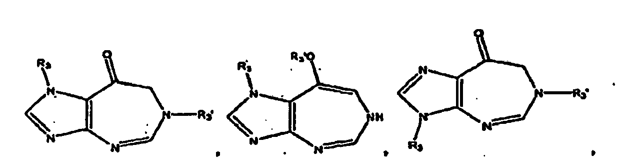 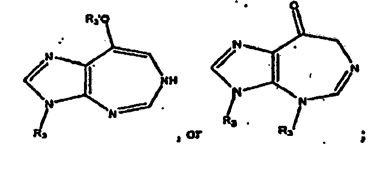
Entschützen des geschützten Diazepinon-Vorläufers, um den Diazepinon-Vörläufer 8a mit der folgenden Formel zu ergeben 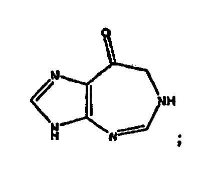
Kondensieren des N-2 des Diazepinon-Vorläufers 8a mit der C-1 Position von 2-Desoxy-D-ribose oder ihrem Derivat, um ein Intermediat 9a mit der folgenden Formel zu ergeben 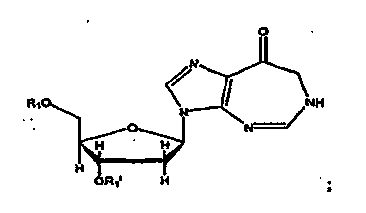 und
Reduzieren der 8-Keto funktionellen Gruppe der Verbindung 9a, um Pentostatin zu ergeben, worin R₁ und R_{1'} jeweils unabhängig H oder eine Schutzgruppe sind, und R₃ und R_{3'} sind jeweils unabhängig H oder eine Schutzgruppe.

20. Das Verfahren gemäß Ansprüchen 18 und 19, worin das organische Lösungsmittel gewählt ist aus Acetonitril, Chlorbenzen, Dichlormethan, Methylcyclohexan, N-Methylpyrrolidon, Nitromethan, Aceton, DMSO, Ethylacetat, Ethylether und Ethylformiat.

21. Das Verfahren gemäß Ansprüchen 18 oder 19, worin das geschützte Hypoxanthin in der Anwesenheit eines Lewis-Säure-Katalysators reagiert wird.

22. Das Verfahren gemäß Anspruch 21, worin der Lewis-Säure-Katalysator gewählt ist aus Trimethylsilyltriflat, BX₃, AlX₃, FeX₃, GaX₃, SbX₅, SnX₄, AsX₅, ZnX₂ und HgX₂, worin X ein Halogen ist.

23. Das Verfahren gemäß Ansprüchen 18 oder 19, worin das geschützte Hypoxanthin unter wasserfreien Bedingungen reagiert wird.

24. Ein Verfahren zur Herstellung von Pentostatin, das folgendes umfasst:
Bereitstellen eines 2'-Desoxyinosinderivats, worin mindestens einer von dem Hypoxanthinsauerstoff, dem Hypoxanthinamidstickstoff, dem 3'-Hydroxylsauerstoff und 5'-Hydroxylsauerstoff durch eine Schutzgruppe geschützt ist;
Erweitern der O-C-N Funktionalität (O=C-NH oder HO-C=N) des 6-gliedrigen Rings des 2-Desoxyinosinderivats, um ein Intermediat mit der Formel 20a oder 20b zu erzeugen 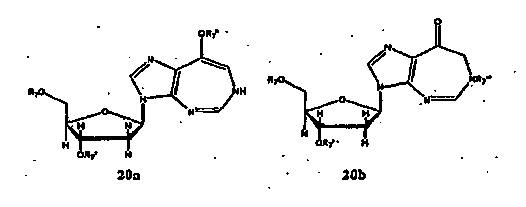 und
Entschützen und Reduzieren der 8-keto funktionellen Gruppe der Verbindung 20a oder 20b, um Pentostatin zu ergeben, worin R₇, R_{7'}, R_{7''} und R_{7'"} jeweils unabhängig H oder eine Schutzgruppe sind.

25. Das Verfahren gemäß Anspruch 24, worin R₇ und R_{7'} jeweils unabhängig eine Schutzgruppe sind, gewählt aus der Gruppe bestehend aus Benzylethern, Silylethern und Estern.

26. Das Verfahren gemäß Anspruch 24, worin R_{7"} eine Schutzgruppe ist, gewählt aus der Gruppe bestehend aus Benzylethern und Silylethern.

27. Das Verfahren gemäß Anspruch 24, worin R_{7'''} eine Carbamat-Schutzgruppe ist.

28. Das Verfahren gemäß Anspruch 24, worin das Erweitern der O-C-N Funktionalität (O=CNH oder HO-C=N) des 6-gliedrigen Rings des 2'-Desoxyinosinderivats das Reagieren des 2'-Desoxyinosinderivats mit Diazomethan oder Trimethylsilyldiazomethan in der Anwesenheit eines Lewis-Säure-Katalysators einschließt.

29. Das Verfahren gemäß Anspruch 27, worin das Reagieren des 2'-Desoxyinsoinderivats mit Diazomethan oder Trimethylsilyldiazomethan das Reagieren des 2'-Deoxyinsoinderivats mit wasserfreier Lösung von Diazomethan oder Trimethylsilyldiazomethan in Ether einschließt.

30. Das Verfahren gemäß Anspruch 24, worin R₇, R_{7'}, R_{7"} und R_{7'''} jeweils unabhängig eine Silylether-Schutzgruppe sind.

31. Das Verfahren gemäß Anspruch 24, worin das Entschützen und Reduzieren der 8-Keto funktionellen Gruppe von Verbindung 20a oder 20b folgendes einschließt:
Entschützen von Verbindung 20a oder 20b, um einen Pentostatin-Vorläufer mit der folgenden Formel herzustellen 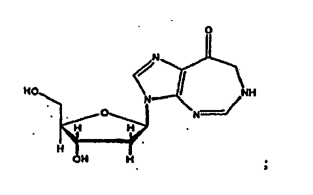 und
Reduzieren der 8-Keto funktionellen Gruppe des Pentostatin-Vorläufers, um Pentostatin zu ergeben.

32. Ein Verfahren zur Herstellung von Coformycin, das folgendes umfasst:
Bereitstellen eines Inosinderivats, worin mindestens einer von dem Hypoxanthin-Sauerstoff, dem Hypoxanthinamid-Stickstoff, dem 2'-Hydroxyl-Sauerstoff, dem 3'-Hydroxyl-Sauerstoff und 5'-Hydroxyl-Sauerstoff geschützt ist durch eine Schutzgruppe;
Erweitern der O-C-N Funktionalität (O=C-NH oder HO-C=N) des 6-gliedrigen Rings des Inosinderivats, um ein Intermediat mit der Formel 21a oder 21b zu erzeugen 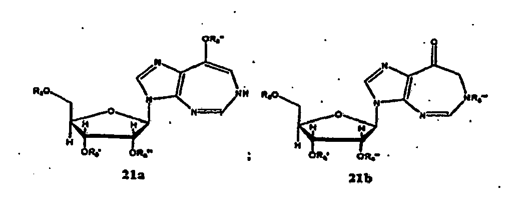 und
Entschützen und Reduzieren der 8-Keto funktionellen Gruppe der Verbindung 21a oder 21b, um Coformycin zu ergeben, worin R₈, R_{8'}, R_{8"}, R_{8"'}, und R_{8""} jeweils unabhängig H oder eine Schutzgruppe sind.

33. Das Verfahren gemäß Anspruch 32, worin R₈, R_{8'} und R_{8"'} jeweils unabhängig eine Schutzgruppe sind, gewählt aus der Gruppe bestehend aus Benyzlethern, Silylethern und Estern.

34. Das Verfahren gemäß Anspruch 33, worin die Benzylether-Schutzgruppe gewählt ist aus der Gruppe bestehend aus p-Methoxybenzyl, 3,4-Dimethoxybenzyl, Nitrobenzyl und p-Cyanobenzyl.

35. Das Verfahren gemäß irgendeinem der Ansprüche 25 und 33, worin die Ester-Schutzgruppe gewählt ist aus der Gruppe bestehend aus Acetat, halogeniertem Acetat, Alkoxyacetat und Benzoat.

36. Das Verfahren gemäß Anspruch 32, worin R_{8"} eine Schutzgruppe ist, gewählt aus der Gruppe bestehend aus Benzylethern und Silylethern.

37. Das Verfahren gemäß irgendeinem der Ansprüche 25, 26 und 36, worin die Benzylether-Schutzgruppe gewählt ist aus der Gruppe bestehend aus p-Methoxybenzyl, 3,4-Dimethoxybenzyl, Nitrobenzyl, und p-Cyanobenzyl.

38. Das Verfahren gemäß irgendeinem der Ansprüche 25, 26, 33 und 36, worin die Silylether-Schutzgruppe gewählt ist aus der Gruppe bestehend aus Trialkylsilyl und Alkoxydialkylsilyl.

39. Das Verfahren gemäß Anspruch 38, worin die Trialkylsilyl-Schutzgruppe gewählt ist aus der Gruppe bestehend aus Trimethylsilyl, Triethylsilyl, Triisopropylsilyl, Dimethylisopropylsilyl, Diethylisopropylsilyl, t-Butyldimethylsilyl, Tribenzylsilyl, Triphenylsilyl, di-t-Butylmethylsilyl und tris(Trimethylsilyl)silyl.

40. Das Verfahren gemäß Anspruch 38, worin die Alkoxydialkylsilyl-Schutzgruppe gewählt ist aus der Gruppe bestehend aus t-Butylmethoxyphenylsilyl und t-Butoxydiphenylsilyl.

41. Das Verfahren gemäß Anspruch 32, worin R_{8""} eine Carbamat-Schutzgruppe ist.

42. Das Verfahren gemäß irgendeinem der Ansprüche 2, 27 und 41, worin die Carbamat-Schutzgruppe gewählt ist aus der Gruppe bestehend aus Methylcarbamat, Ethylcarbamat, t-Butylcarbamat, Benzylcarbamat, 9-Fluorenylmethylcarbamat, 2,2,2-Trichlorethylcarbamat, 1-Methyl-1-(4-biphenyl) Ethylcarbamat und 1-(3,5-di-t-Butyl)-1-methylethylcarbamat.

43. Das Verfahren gemäß Anspruch 32, worin das Erweitern des 6-gliedrigen Rings des Inosinderivats das Reagieren des Insoinderivats mit Diazomethan oder Trimethylsilyldiazomethan in der Anwesenheit eines Lewis-Säure-Katalysators einschließt.

44. Das Verfahren gemäß irgendeinem der Ansprüche 15, 28 und 43, worin der Lewis-Säure-Katalysator gewählt ist aus der Gruppe bestehend aus Trimethylsilyltriflat (TMSOTf), BX₃, AIX₃, FeX₃, GaX₃, SbX₅, SnX₄, AsX₅, ZnX₂ und HgX₂, worin X ein Halogen ist.

45. Das Verfahren gemäß irgendeinem der Ansprüche 15, 28 und 43, worin der Lewis-Säure-Katalysator BF₃-Et₂O, ZnCl₂ oder HgBr₂ ist.

46. Das Verfahren gemäß Anspruch 43, worin das Reagieren des Inosinderivats mit Diazomethan oder Trimethylsilyldiazomethan, das Reagieren des Hypoxanthinderivats mit wasserfreier Lösung von Diazomethan oder Trimethylsilyldiazomethan in Ether einschließt.

## Revendications

1. Procédé de préparation de pentostatine, consistant à :
fournir un dérivé d'hypoxanthine dans lequel au moins une de l'amine secondaire imidazole et de la fonctionnalité O-C-N (O=C-NH ou HO-C=N) est protégée par un groupe protecteur ;
agrandir le cycle de 6 éléments du dérivé d'hypoxanthine pour former un précurseur de diazépinone protégé répondant à la formule 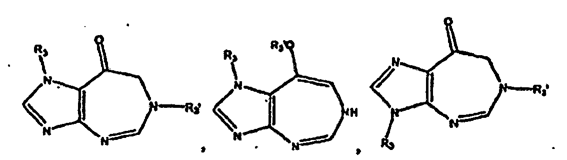 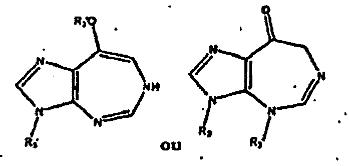
déprotéger le précurseur de diazépinone protégé pour donner un précurseur de diazépinone 8a répondant à la formule 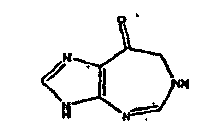
condenser le N-2 du précurseur de diazépinone 8a avec le C-1 du 2-désoxy-D-ribose ou de son dérivé pour donner un intermédiaire répondant à la formule 9a 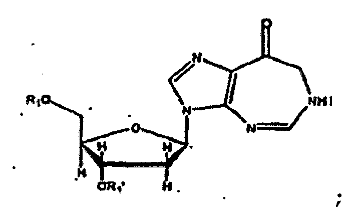 et
réduire le groupe fonctionnel 8-céto du composé 9a pour donner la pentostatine,
où R₁ et R_{1'} sont chacun indépendamment H ou un groupe protecteur, et R₃ et R_{3'} sont chacun indépendamment H ou un groupe protecteur.

2. Procédé selon la revendication 1, dans lequel R₃ et R_{3'} sont chacun indépendamment un groupe protecteur de type carbamate, amide, arylamine et silylamine.

3. Procédé selon la revendication 2, dans lequel le groupe protecteur de type amide est choisi dans le groupe constitué par l'acétamide, le trifluoroacétamide et le benzamide.

4. Procédé selon la revendication 2, dans lequel le groupe protecteur de type arylamine est choisi dans le groupe constitué par la benzylamine, la 4-méthoxybenzylamine et la 2-hydroxybenzylamine.

5. Procédé selon la revendication 1, dans lequel R₁ et R₁, sont chacun indépendamment un groupe protecteur choisi dans le groupe constitué par un éther, un ester, un carbonate, un sulfonate, un acétal cyclique et un cétal, une cétone chirale, un orthoester cyclique, un dérivé silylique, un carbonate cyclique et un borate cyclique.

6. Procédé selon la revendication 1, dans lequel R₁ et R_{1'} sont chacun indépendamment un groupe protecteur de type p-toluoyle.

7. Procédé selon la revendication 5, dans lequel le groupe protecteur de type éther est choisi dans le groupe constitué par les groupes méthoxyméthyle, benzyloxyméthyle, allyle, propargyle, p-chlorophényle, p-méthoxyphényle, p-nitrophényle, benzyle, p-méthoxybenzyle, diméthoxybenzyle, nitrobenzyle, benzyle halogéné, cyanobenzyle, triméthylsilyle, triméthylsilyle, triisopropylsilyle, tribenzylsilyle et alcoxysilyle.

8. Procédé selon la revendication 5, dans lequel le groupe protecteur de type ester est choisi dans le groupe constitué par les acétates et les benzoates.

9. Procédé selon la revendication 5, dans lequel le groupe protecteur de type carbonate est choisi dans le groupe constitué par les groupes méthoxyméthyle, 9-fluorénylméthyle, 2,2,2-trichloroéthyle, vinyle, allyle, nitrophényle et benzyle.

10. Procédé selon la revendication 5, dans lequel le groupe protecteur de type sulfonate est choisi dans le groupe constitué par les groupes allylsulfonate, mésylate, benzylsulfonate et tosylate.

11. Procédé selon la revendication 5, dans lequel le groupe protecteur de type acétal cyclique ou cétal est choisi dans le groupe constitué par les groupes méthylène, éthylidène, acroléine, isopropylidène, cyclopentylidène, cyclohexylidène, cycloheptylidène, benzylidène, mésitylène, 1-naphtaldéhyde acétal, benzophénone cétal et éther o-xylylique.

12. Procédé selon la revendication 5, dans lequel le groupe protecteur de type cétone chirale est choisi dans le groupe constitué par les groupes camphre et menthone.

13. Procédé selon la revendication 5, dans lequel le groupe protecteur de type orthoester cyclique est choisi dans le groupe constitué par les groupes méthoxyméthylène, éthoxyméthylène, 1-méthoxyéthylidène, méthylidène, phtalide, dérivés de l'éthylidène et du benzylidène, butane-2,3-bisacétal, cyclohexane-1,2-diacétal et dispirocétal.

14. Procédé selon la revendication 5, dans lequel le groupe protecteur de type dérivé silylique est choisi dans le groupe constitué par les groupes di-t-butylsilylène et dialkylsilylène.

15. Procédé selon la revendication 1, dans lequel l'agrandissement du cycle de 6 éléments du dérivé d'hypoxanthine consiste à faire réagir le dérivé d'hypoxanthine avec du diazométhane ou du triméthylsilyldiazométhane en présence d'un catalyseur de type acide de Lewis.

16. Procédé selon la revendication 15, dans lequel la mise en réaction du dérivé d'hypoxanthine avec du diazométhane ou du triméthylsilyldiazométhane comprend la mise en réaction du dérivé d'hypoxanthine avec une solution anhydre de diazométhane ou de triméthylsilyldiazométhane dans de l'éther.

17. Composé qui est un précurseur de pentostatine ou d'autres composés hétérocycliques répondant à la formule 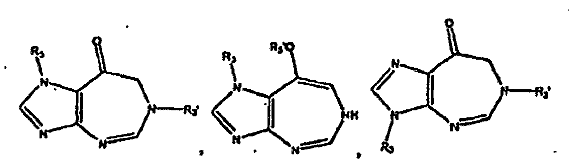 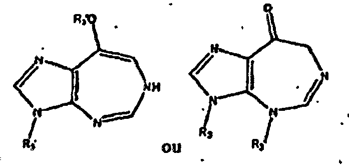 où R₃ et R_{3'} sont chacun indépendamment H ou un groupe protecteur, et au moins un de R₃ et R_{3'} est un groupe protecteur,
où le groupe protecteur est choisi parmi un groupe protecteur de type carbamate, un groupe protecteur de type amide, un groupe protecteur de type arylamine ou un groupe protecteur de type silylamine,
où le groupe protecteur de type carbamate est choisi dans le groupe constitué par les groupes carbamate de méthyle, carbamate d'éthyle, carbamate de t-butyle, carbamate de benzyle, carbamate de 9-fluoroénylméthyle, carbamate de 2,2,2-trichloroéthyle, carbamate de 1-méthyl-1-(4-biphényl)éthyle et carbamate de 1-(3,5-di-t-butyl)-1-méthyléthyle :
où le groupe protecteur de type amide est choisi dans le groupe constitué par les groupes acétamide, trifluoroacétamide et benzamide ;
où le groupe protecteur de type arylamine est choisi dans le groupe constitué par les groupes benzylamine, 4-méthoxybenzylamine et 2-hydroxybenzylamine ; et
où le groupe protecteur de type silyle est choisi dans le groupe constitué par les groupes di-t-butylsilylène, dialkylsilylène, trialkylsilyle choisis parmi les groupes triméthylsilyle, triéthylsilyle, triisopropylsilyle, diméthylisopropylsilyle, diéthylisopropylsilyle, t-butyldiméthylsilyle, tribenzylsilyle, triphénylsilyle, di-t-butylméthylsilyle et tris(triméthylsilyl)silyle et alcoxydialkylsilyle choisis parmi les groupes t-butylméthoxyphénylsilyle et t-butoxydiphénylsilyle.

18. Procédé de fabrication d'un précurseur de diazépinone de pentostatine constituant à
protéger l'hypoxanthine en un ou plusieurs endroits en utilisant un groupe protecteur ;
faire réagir l'hypoxanthine protégée dans un solvant organique pour donner un précurseur de diazépinone protégé répondant à la formule 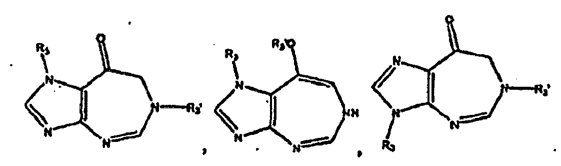 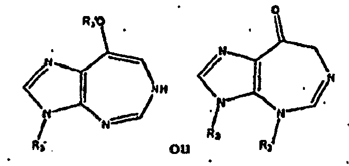 où R₃ et R_{3'} sont chacun indépendamment H ou un groupe protecteur ;
faire précipiter le précurseur de diazépinone protégé, et
déprotéger le précurseur de diazépinone protégé pour donner le précurseur de diazépinone 8a répondant à la formule

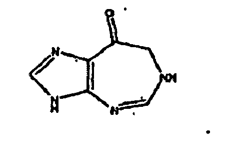

19. Procédé de fabrication de pentostatine, consistant à :
protéger l'hypoxanthine en un ou plusieurs endroits en utilisant un groupe protecteur ;
faire réagir l'hypoxanthine protégée dans un solvant organique pour donner un précurseur de diazépinone protégé répondant à la formule 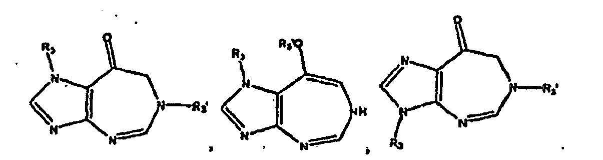 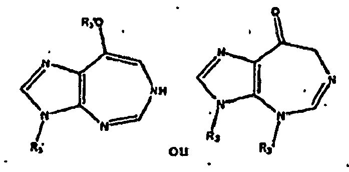
déprotéger le précurseur de diazépinone protégé pour donner le précurseur de diazépinone 8a répondant à la formule 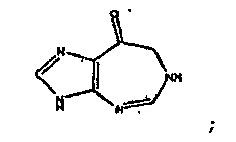
condenser le N-2 du précurseur de diazépinone 8a avec la position C-1 du 2-désoxy-D-ribose ou de son dérivé pour donner un intermédiaire 9a répondant à la formule 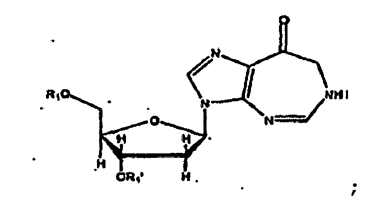 et
réduire le groupe fonctionnel 8-céto du composé 9a pour donner la pentostatine,
où R₁ et R_{1'} sont chacun indépendamment H ou un groupe protecteur, et R₃ et R_{3'} sont chacun indépendamment H ou un groupe protecteur.

20. Procédé selon la revendication 18 ou 19, dans lequel le solvant organique est choisi parmi l'acétonitrile, le chlorobenzène, le dichlorométhane, le méthylcyclohexane, la N-méthylpyrrolidone, le nitrométhane, l'acétone, le DMSO, l'acétate d'éthyle, l'éther éthylique et le formiate d'éthyle.

21. Procédé selon la revendication 18 ou 19, dans lequel l'hypoxanthine protégée est mise à réagir en présence d'un catalyseur de type acide de Lewis.

22. Procédé selon la revendication 21, dans lequel le catalyseur de type acide de Lewis est choisi parmi le triflate de triméthylsilyle, BX₃, AlX₃, FeX₃, GaX₃, SbX₅, SnX₄, AsX₅, ZnX₂ et HgX₂, où X est un atome d'halogène.

23. Procédé selon la revendication 18 ou 19, dans lequel l'hypoxanthine protégée est mise à réagir dans des conditions anhydres.

24. Procédé de préparation de pentostatine consistant à :
fournir un dérivé de 2'-désoxyinosine dans lequel au moins un de l'oxygène de l'hypoxanthine, l'azote de l'amide de l'hypoxanthine, l'oxygène du 3'-hydroxyle et l'oxygène du 5'-hydroxyle est protégé par un groupe protecteur ;
agrandir la fonctionnalité O-C-N (O=C-N ou OH-C=N) du cycle de 6 éléments du dérivé 2'-désoxyinosine pour produire un intermédiaire répondant à la formule 20a ou 20b 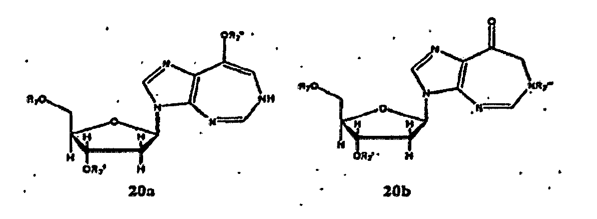 et
déprotéger et réduire le groupe fonctionnel 8-céto du composé 20a ou 20b pour donner la pentostatine, où R₇, R_{7'}, R_{7''} et R_{7'''} sont chacun indépendamment H ou un groupe protecteur.

25. Procédé selon la revendication 24, dans lequel R₇ et R_{7'} sont chacun indépendamment un groupe protecteur choisi dans le groupe constitué par les éthers benzyliques, les éthers silyliques et les esters.

26. Procédé selon la revendication 24, dans lequel R_{7''} est un groupe protecteur qui est choisi dans le groupe constitué par les éthers benzyliques et les éthers silyliques.

27. Procédé selon la revendication 24, dans lequel R_{7'''} est un groupe protecteur de type carbamate.

28. Procédé selon la revendication 24, dans lequel l'agrandissement de la fonctionnalité O-C-N (O=C-N ou OH-C=N) du cycle de 6 éléments du dérivé de 2'-désoxyinosine consiste à faire réagir le dérivé de 2'-désoxyinosine avec du diazométhane ou du triméthylsilyldiazométhane en présence d'un catalyseur de type acide de Lewis.

29. Procédé selon la revendication 27, dans lequel la mise en réaction du dérivé de 2'-désoxyinosine avec du diazométhane ou du triméthylsilyldiazométhane consiste à faire réagir le dérivé de 2'-désoxyinosine avec une solution anhydre de diazométhane ou de triméthylsilyldiazométhane dans de l'éther.

30. Procédé selon la revendication 24, dans lequel R₇, R_{7'}, R_{7''} et R_{7'''} sont chacun indépendamment un groupe protecteur de type éther silylique.

31. Procédé selon la revendication 24, dans lequel la déprotection et la réduction le groupe fonctionnel 8-céto du composé 2a ou 20b consistent à
déprotéger le composé 20a ou 20b pour produire un précurseur de pentostatine répondant à la formule suivante 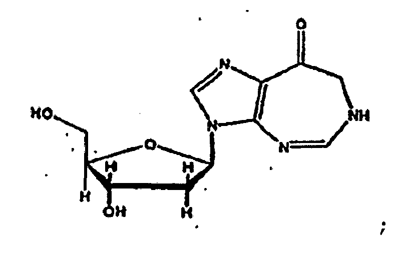 et
réduire le groupe fonctionnel 8-céto du précurseur de pentostatine pour donner la pentostatine.

32. Procédé de préparation de coformycine, consistant à :
fournir un dérivé d'inosine dans lequel au moins un de l'oxygène de l'hypoxanthine, l'azote de l'amide de l'hypoxanthine, l'oxygène du 2'-hydroxyle, l'oxygène du 3'-hydroxyle et l'oxygène du 5'-hydroxyle est protégé par un groupe protecteur ;
agrandir la fonctionnalité O-C-N (O=C-N ou OH-C=N) du cycle de 6 éléments du dérivé d'inosine pour produire un intermédiaire répondant à la formule 21a ou 21b 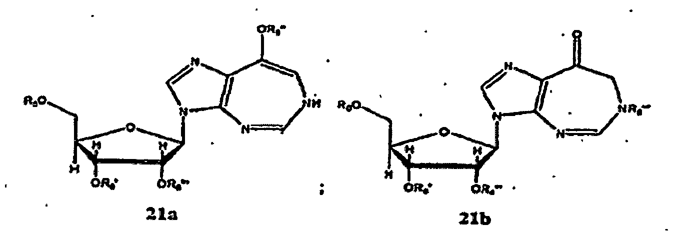 et
déprotéger et réduire le groupe fonctionnel 8-céto du composé 21a ou 21b pour donner la coformycine, où R₈, R_{8'}, R_{8"}, R_{8"'} et R_{8""} sont chacun indépendamment H ou un groupe protecteur.

33. Procédé selon la revendication 32, dans lequel R₈, R_{8'} et R_{8"'} sont chacun indépendamment un groupe protecteur choisi dans le groupe constitué par les éthers benzyliques, les éthers silyliques et les esters.

34. Procédé selon la revendication 33, dans lequel le groupe protecteur de type éther benzylique est choisi dans le groupe constitué par les groupes p-méthoxybenzyle, 3,4-diméthoxybenzyle, nitrobenzyle et p-cyanobenzyle.

35. Procédé selon l'une quelconque des revendications 25 et 33, dans lequel le groupe protecteur ester est choisi dans le groupe constitué par les groupes acétate, acétate halogéné, alcoxyacétate et benzoate.

36. Procédé selon la revendication 32, dans lequel R_{8"} est un groupe protecteur qui est choisi dans le groupe constitué par les éthers benzyliques et les éthers silyliques.

37. Procédé selon l'une quelconque des revendications 25, 26 et 36, dans lequel le groupe protecteur de type éther benzylique est choisi dans le groupe constitué par les groupes p-méthoxybenzyle, 3,4-diméthoxybenzyle, nitrobenzyle et p-cyanobenzyle.

38. Procédé selon l'une quelconque des revendications 25, 26, 33 et 36, dans lequel le groupe protecteur de type éther silylique est choisi dans le groupe constitué par les groupes trialkylsilyle et alcoxydialkylsilyle.

39. Procédé selon la revendication 38, dans lequel le groupe protecteur de type trialkylsilyle est choisi dans le groupe constitué par les groupes triméthylsilyle, triéthylsilyle, triisopropylsilyle, diméthylisopropylsilyle, diéthylisopropylsilyle, t-butyldiméthylsilyle, tribenzylsilyle, triphénylsilyle, di-t-butylméthylsilyle et tris(triméthylsilyl)silyle.

40. Procédé selon la revendication 38, dans lequel le groupe protecteur alcoxydialkylsilyle est choisi dans le groupe constitué par les groupes t-butylméthoxyphénylsilyle et t-butoxydiphénylsilyle.

41. Procédé selon la revendication 32, dans lequel R_{8""} est un groupe protecteur de type carbamate.

42. Procédé selon l'une quelconque des revendications 2, 27 et 41, dans lequel le groupe protecteur de type carbamate est choisi dans le groupe constitué par les groupes carbamate de méthyle, carbamate d'éthyle, carbamate de t-butyle, carbamate de benzyle, carbamate de 9-fluorénylméthyle, carbamate de 2,2,2-trichloroéthyle, carbamate de 1-méthyl-1-(4-biphényl)éthyle et carbamate de 1-(3,5-di-t-butyl)-1-méthyléthyle.

43. Procédé selon la revendication 32, dans lequel l'agrandissement du cycle de 6 éléments du dérivé d'inosine consiste à faire réagir le dérivé d'inosine avec du diazométhane ou du triméthylsilyldiazométhane en présence d'un catalyseur de type acide de Lewis.

44. Procédé selon l'une quelconque des revendications 15, 28 et 43, dans lequel le catalyseur de type acide de Lewis est choisi dans le groupe constitué par le triflate de triméthylsilyle (MSOTf), BX₃, AlX₃, FeX₃, GaX₃, SbX₅, SnX₄, AsX₅, ZnX₂ et HgX₂, où X est un atome d'halogène.

45. Procédé selon l'une quelconque des revendications 15, 28 et 43, dans lequel le catalyseur de type acide de Lewis est BF₃-Et₂O, ZnCl₂ ou HgBr₂.

46. Procédé selon la revendication 43, dans lequel la mise en réaction du dérivé d'inosine avec du diazométhane ou du triméthylsilyldiazométhane consiste à faire réagir le dérivé d'hypoxanthine avec une solution anhydre de diazométhane ou de triméthylsilyldiazométhane dans de l'éther.
